(19) Europäisches Patentamt / European Patent Office / Office européen des brevets

(11) **EP 2 930 191 B1**

(12) **EUROPEAN PATENT SPECIFICATION**

(45) Date of publication and mention
of the grant of the patent:
**16.09.2020 Bulletin 2020/38**

(21) Application number: **14163666.2**

(22) Date of filing: **07.04.2014**

(51) Int Cl.:
*C08F 2/44* (2006.01)  *A61L 15/22* (2006.01)
*A61L 15/60* (2006.01)  *B01J 20/00* (2006.01)
*C08J 3/12* (2006.01)  *C08F 220/06* (2006.01)
*C08J 9/00* (2006.01)  *C08J 3/24* (2006.01)
*A61L 27/12* (2006.01)  *A61L 27/58* (2006.01)
*A61L 15/42* (2006.01)  *B01J 20/12* (2006.01)
*B01J 20/26* (2006.01)  *C08J 9/08* (2006.01)
*C08J 9/20* (2006.01)  *B01J 20/32* (2006.01)
*C08J 9/224* (2006.01)  *C08F 220/18* (2006.01)

(54) **Superabsorbent polymer having fast absorption**

Hochsaugfähiges Polymer mit schneller Absorption

Polymère super-absorbant à absorption rapide

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(43) Date of publication of application:
**14.10.2015 Bulletin 2015/42**

(73) Proprietor: **Evonik Corporation
Parsippany NJ 07054 (US)**

(72) Inventors:
• **Azad, Michael**
**Reidsville, NC North Carolina 27320 (US)**
• **Wieand, Rani**
**Hillsborough, NC North Carolina 27278 (US)**
• **Gilmer, Regina**
**Greensboro, NC North Carolina 27406 (US)**
• **Blake, Geoffrey W.**
**Kernersville, NC 27284 (US)**
• **Wiedbusch, Ron**
**Greensboro, NC 27455 (US)**
• **Bergman, David L. jr.**
**Greensboro, NC 27410 (US)**
• **Lang, Andrew J.**
**High Point, NC 27265 (US)**

(74) Representative: **Evonik Patent Association
c/o Evonik Industries AG
IP Management
Bau 1042A/PB 15
Paul-Baumann-Straße 1
45772 Marl (DE)**

(56) References cited:
**WO-A1-02/053198**   **WO-A1-2010/057912**
**WO-A1-2013/076031**

**Description**

The invention relates to superabsorbent polymers which absorb water, aqueous liquids and blood wherein the superabsorbent polymers of the present invention has fast absorption. The present invention also relates to preparation of these superabsorbent polymers having fast absorption and their use as absorbents in hygiene articles.

A superabsorbent polymer, in general refers to a water-swellable, water-insoluble polymer, or material, capable of absorbing at least about 10 times its weight, and up to about 30 times or more its weight in an aqueous solution containing 0.9 weight percent sodium chloride solution in water. Examples of superabsorbent polymer may include a crosslinked partially neutralized acrylate polymer, and the formation of superabsorbent polymer hydrogel from the polymerization, and formation of particulate superabsorbent polymer compositions capable of retaining the aqueous liquids under a certain pressure in accordance with the general definition of superabsorbent polymer.

The superabsorbent polymer hydrogel can be formed into particles, generally referred to as particulate superabsorbent polymer, wherein the particulate superabsorbent polymer may be surface-treated with surface crosslinking, and other surface treatment and post treated after surface crosslinking to form particulate superabsorbent polymer compositions. The acronym SAP may be used in place of superabsorbent polymer, superabsorbent polymer composition, particulate superabsorbent polymer compositions, or variations thereof. In general, these particulate superabsorbent polymer compositions have a centrifuge retention capacity (CRC) of at least 25 grams of 0.9 weight percent sodium chloride aqueous solution per gram of the polymer. Particulate superabsorbent polymer compositions are also designed to quickly uptake bodily fluids, which require high gel bed permeability (GBP).

Commercial particulate superabsorbent polymer compositions are widely used in a variety of personal care products, such as infant diapers, child training pants, adult incontinence products, feminine care products, and the like.

Speed of absorption and increase of the speed is one aspect of superabsorbent polymer. In field of superabsorbents, speed of absorption can be quantified by measuring the so called "vortex" time. Fast superabsorbents are achieving low vortex times.

WO02053198A1 discloses a water-absorbent composition containing, in relation to its weight, between 30 and 100 wt. % of non-water soluble hydrogels which can swell in water. Said hydrogels comprises the following characteristics: a centrifuge retention capacity (CRC) of at least 24 g/g; a saline flow conductivity (SFC) of at least $80*10^{-7}$ $cm^3s/g$; and a free swell rate (FSR) of at least 0.15 g/g and/or a maximum vortex time of 160s. Some examples showing vortex times below 60s.

WO2010057912A1 discloses a method for producing water-absorbing polymer particles through polymerization of drops of a monomer solution in a surrounding gas phase, wherein the polymer particles are coated with a permeability improving agent. Examples showing vortex times below 60s.

WO2013076031A1 discloses a superabsorbent polymer with pyrogenium aluminum oxide having low caking tendency with good absorption properties and rapid water absorption. Examples showing vortex times below 60s.

Adding a foaming agent such as sodium carbonate or sodium bicarbonate to the superabsorbent polymer before polymerization has been disclosed as one way to increase the speed of absorption of the superabsorbent polymer. Using a foaming agent results in a method for pyrolytically forming bubbles in the superabsorbent polymer and relies on the heat generated by polymerization to start the foaming. This foaming method has such problems as the polymerization in process resulting in a large change in volume, allowing no easy control, and producing a polymer lacking homogeneity in quality and having a particularly high content of water-insoluble components and the produced foam lacking stability of pore diameter and distribution and allowing now sufficient control of absorbing speed.

When this method is carried out by the use of an azo based polymerization initiator, the amount of this initiator is generally increased to form bubbles in an amount enough to improve the absorbing speed and consequently the content of water-soluble components in the produced polymer tends to increase. Further, the method using the azo based polymerization initiator, similarly to the method using the foaming agent, has the problem that the polymerization in process will result in a large change of volume and will consequently allow no easy control of pore particle and distribution of bubbles.

When this method implements the polymerization in the presence of dispersion of such a water-insoluble foaming agent as a volatile organic compound, though the polymerization can be effected relatively stability, the method incurs heavy waste of energy, proves expensive, and lacks practical serviceability because the polymerization requires a special apparatus from the viewpoint of safety on account of the use of the volatile organic compound and the used volatile organic compound is discharged from the system.

These particulate superabsorbent polymers, however, are invariably at a disadvantage in showing no sufficient absorbing speed without and under load, allowing no easy drying, suffering a large load during the pulverization, lacking uniformity of pore diameter, and having a large content of water-soluble components.

An object of this invention, therefore, is to provide a particulate superabsorbent polymer capable of fast absorption of water and a method for the production thereof. It is therefore an object of the present invention to provide a superabsorbent polymer that exhibits increased rate of water absorption as well as maintaining excellent properties.

The present invention has realized a water-absorbent resin which allows production of a foam uniform in distribution of bubbles, permits fast absorption of water, and a method for the production of thereof.

The present invention is directed to a superabsorbent polymer comprising an foaming agent, lipophile nonionic surfactant and a polyethoxylated hydrophilic nonionic surfactant wherein the particulate superabsorbent polymer has a vortex time of 30 to 60 seconds.

The present invention is also directed to a particulate superabsorbent polymer comprising having an internal crosslinking structure, from about 0.05 to about 2.0 wt.% of a foaming agent, and from about 0.001 to about 1.0 wt.% of a mixture of a lipophile surfactant and a polyethoxylated hydrophilic surfactant, the particle having a surface which has been subjected to a cross-linking treatment for cross-linking the surface, the particulate superabsorbent polymer having a vortex time of from 30 to 60 seconds.

The present invention is also directed to a process for making a particulate superabsorbent polymer having fast water absorption comprising the steps of

a) preparing an aqueous monomer solution of a mixture of a of polymerizable unsaturated acid group containing monomer and an internal crosslinking agent monomer wherein the aqueous monomer solution comprises dissolved oxygen;

b) sparging the aqueous monomer solution of step a) including adding an inert gas to the aqueous monomer solution of step a) to replace the dissolved oxygen of the aqueous monomer solution;

c) polymerizing the aqueous monomer solution of step b) including the steps of

c1) adding to the aqueous monomer solution of step a): i) an aqueous solution comprising from about 0.1 to about 1.0 wt.% based on the total amount of the polymerizable unsaturated acid group containing monomer solution of a foaming agent; and ii) an aqueous solution comprising from about 0.001 to about 1.0 wt.% based on the total amount of the polymerizable unsaturated acid group containing monomer solution of a mixture of a lipophile surfactant and a polyethoxylated hydrophilic surfactant;

c2) treating the monomer solution of step c1) to high speed shear mixing to form a treated monomer solution, wherein the components i) an aqueous solution comprising from about 0.05 to about 2.0 wt.% of the foaming agent; and ii) an aqueous solution comprising from about 0.001 to about 1.0 wt.% of a mixture of the lipophile surfactant and the polyethoxylated hydrophilic surfactant are added to the aqueous monomer solution after step b) of sparging the aqueous monomer solution and before step c2) of high speed shear mixing of the aqueous monomer solution;

c3) forming a hydrogel by adding a polymerization initiator to the treated monomer solution of step c2) wherein the initiator is added to the treated monomer solution after the foaming agent and the mixture of surfactants, wherein the polymer is formed to include bubbles of the foaming agent into the polymer structure; and

d) drying and grinding the hydrogel of step c) to form particulate superabsorbent polymer; and

e) surface crosslinking the particulate superabsorbent polymer of step d) with a surface crosslinking agent wherein the surface crosslinked superabsorbent polymer has a vortex time of from about 30 sec to about 60 sec.

With the foregoing in mind, it is a feature and advantage of the invention to provide particulate superabsorbent polymer composition and methods of increasing the speed of water absorption of particulate superabsorbent polymer composition. Numerous other features and advantages of the present invention will appear from the following description.

FIG 1 is a side view of the test apparatus employed for the Free Swell Gel Bed Permeability Test;
FIG 2 is a cross-sectional side view of a cylinder/cup assembly employed in the Free Swell Gel Bed Permeability Test apparatus shown in FIG 1;
FIG 3 is a top view of a plunger employed in the Free Swell Gel Bed Permeability Test apparatus shown in FIG 1;
FIG 4 is a side view of the test apparatus employed for the Absorbency Under Load Test; and
FIG 5 representatively shows a partially cut away, top plan view of an absorbent article in a stretched and laid flat condition with the surface of the article that contacts the skin of the wearer facing the viewer;

Within the context of this specification, each term or phrase below will include the following meaning or meanings.

The term "Centrifuge Retention Capacity (CRC)" as used herein refers to the ability of the particulate superabsorbent polymer to retain liquid therein after being saturated and subjected to centrifugation under controlled conditions and is stated as grams of liquid retained per gram weight of the sample (g/g) as measured by the Centrifuge Retention Capacity Test set forth herein.

The terms "crosslinked", "crosslink", "crosslinker", or "crosslinking" as used herein refers to any means for effectively rendering normally water-soluble materials substantially water-insoluble but swellable. Such a crosslinking means can include, for example, physical entanglement, crystalline domains, covalent bonds, ionic complexes and associations, hydrophilic associations such as hydrogen bonding, hydrophobic associations, or Van der Waals forces.

The term "internal crosslinker" or "monomer crosslinker" as used herein refers to use of a crosslinker in the monomer solution to form the polymer.

The term "dry particulate superabsorbent polymer composition" as used herein generally refers to the superabsorbent polymer composition having less than about 20% moisture.

The term "gel permeability" is a property of the mass of particles as a whole and is related to particle size distribution, particle shape, and the connectedness of the open pores between the particles, shear modulus, and surface modification of the swollen gel. In practical terms, the gel permeability of the superabsorbent polymer composition is a measure of how rapidly liquid flows through the mass of swollen particles. Low gel permeability indicates that liquid cannot flow readily through the superabsorbent polymer composition, which is generally referred to as gel blocking, and that any forced flow of liquid (such as a second application of urine during use of the diaper) must take an alternate path (e.g., diaper leakage).

The acronym "HLB" means the hydrophilic-lipophilic balance of a surfactant and is a measure of the degree to which it is hydrophilic or lipophilic, as determined by calculating values for the different regions of the molecule. The HLB value can be used to predict the surfactant properties of a molecule wherein a HLB value < 10 is lipid soluble (water insoluble) and a HLB value > 10 is water soluble (lipid insoluble).

The term "mass median particle size" of a given sample of particles of superabsorbent polymer composition is defined as the particle size, which divides the sample in half on a mass basis, i.e., half of the sample by weight has a particle size greater than the mass median particle size, and half of the sample by mass has a particle size less than the mass median particle size. Thus, for example, the mass median particle size of a sample of superabsorbent polymer composition particles is 2 microns if one-half of the samples by weight are measured as more than 2 microns.

The term "moisture content" when used herein shall mean the quantity of water contained in the particulate superabsorbent polymer composition as measured by the Moisture Content Test.

The term "non-ionic surfactants" is a surfactant having no charge and can greatly reduce the surface tension of water when used in very low concentrations. They do not ionize in aqueous solutions because their hydrophilic group is of a non-dissociable.

The terms "particle," "particulate," and the like, when used with the term "superabsorbent polymer," refer to the form of discrete units. The units can comprise flakes, fibers, agglomerates, granules, powders, spheres, pulverized materials, or the like, as well as combinations thereof. The particles can have any desired shape: for example, cubic, rod like polyhedral, spherical or semi-spherical, rounded or semi-rounded, angular, irregular, et cetera.

The terms "particulate superabsorbent polymer" and "particulate superabsorbent polymer composition" refer to the form of superabsorbent polymer and superabsorbent polymer compositions in discrete form, wherein the "particulate superabsorbent polymer" and "particulate superabsorbent polymer compositions" may have a particle size of less than 1000μm, or from about 150μm to about 850μm.

The term "permeability", when used herein shall mean a measure of the effective connectedness of a porous structure, in this case, crosslinked polymers, and may be specified in terms of the void fraction, and extent of connectedness of the particulate superabsorbent polymer composition.

The term "polymer" includes, but is not limited to, homopolymers, copolymers, for example, block, graft, random, and alternating copolymers, terpolymers, etc., and blends and modifications thereof. Furthermore, unless otherwise specifically limited, the term "polymer" shall include all possible configurational isomers of the material. These configurations include, but are not limited to isotactic, syndiotactic, and atactic symmetries.

The term "polyolefin" as used herein generally includes, but is not limited to, materials such as polyethylene, polypropylene, polyisobutylene, polystyrene, ethylene vinyl acetate copolymer, and the like, the homopolymers, copolymers, terpolymers, etc., thereof, and blends and modifications thereof. The term "polyolefin" shall include all possible structures thereof, which include, but are not limited to, isotatic, synodiotactic, and random symmetries. Copolymers include atactic and block copolymers.

The term "superabsorbent polymer" as used herein refers to water-swellable, water-insoluble organic or inorganic materials including superabsorbent polymers and superabsorbent polymer compositions capable, under the most favorable conditions, of absorbing at least about 10 times their weight, or at least about 15 times their weight, or at least about 25 times their weight in an aqueous solution containing 0.9 weight percent sodium chloride.

The term "superabsorbent polymer composition" as used herein refers to a superabsorbent polymer comprising a surface additive in accordance with the present invention.

The term "surface crosslinking" as used herein refers to the level of functional crosslinks in the vicinity of the surface of the superabsorbent polymer particle, which is generally higher than the level of functional crosslinks in the interior of the superabsorbent polymer particle. As used herein, "surface" describes the outer-facing boundaries of the particle.

The term "thermoplastic" as used herein describes a material that softens when exposed to heat and which substantially returns to a non-softened condition when cooled to room temperature.

The term "vortex time" measures the amount of time in seconds required for 2 grams of a SAP to close a vortex created by stirring 50 milliliters of saline solution at 600 revolutions per minute on a magnetic stir plate. The time it takes for the vortex to close is an indication of the free swell absorbing rate of the SAP.

The term "% by weight" or "%wt" as used herein and referring to components of the dry particulate superabsorbent polymer composition, is to be interpreted as based on the weight of the dry superabsorbent polymer composition, unless otherwise specified herein.

These terms may be defined with additional language in the remaining portions of this specification including the following Detailed Description.

In accordance with the invention, a particulate superabsorbent polymer composition having fast absorption can be achieved using the methods described herein. The present invention further includes an absorbent article comprising a topsheet; a backsheet; and an absorbent core disposed between the topsheet and backsheet, the absorbent core comprising the various embodiments of the particulate superabsorbent polymer composition of this invention.

The present invention is also directed to a particulate superabsorbent polymer comprising having an internal crosslinking structure, from about 0.05 to about 2.0 wt.% of a foaming agent, and from about 0.001 to about 1.0 wt.% of a mixture of a lipophile surfactant and a polyethoxylated hydrophilic surfactant in an inside of the particle, the particle having a surface which has been subjected to a cross-linking treatment for cross-linking the surface, the particulate superabsorbent polymer having a vortex time of from 30 to 60 seconds.

The present invention is also directed to a particulate superabsorbent polymer wherein the lipophile surfactant has a HLB of from 4 to 9 and the polyethoxylated hydrophilic surfactant has a HLB of from 12 to 18; or wherein the mixture of a lipophile surfactant and a polyethoxylated hydrophilic surfactant has a HLB of from 8 to 14; or wherein the lipophile surfactant is a sorbitan ester and the polyethoxylated hydrophilic surfactant is a polyethoxylated sorbitan ester; or wherein the lipophile surfactant is a nonionic and the polyethoxylated hydrophilic surfactant is nonionic; or wherein the foaming agent is selected from an alkali metal carbonate or alkali metal bicarbonate wherein the alkali metal may be sodium or potassium; or wherein the superabsorbent polymer has a Pressure Absorbency Index of from about 120 to about 150; or wherein the internal crosslinker agent comprises a silane compound comprising at least one vinyl group or allyl group and at least one Si-O bond wherein the vinyl group or allyl group is directly attached to a silicon atom.

The present invention is also directed to a particulate superabsorbent polymer comprising having an internal crosslinking structure, from about 0.05 to about 2.0 wt.% of a foaming agent, and from about 0.001 to about 1.0 wt.% of a mixture of a lipophile nonionic surfactant and a polyethoxylated hydrophilic nonionic surfactant in an inside of the particle, the particle having a surface which has been subjected to a cross-linking treatment for cross-linking the surface, the particulate superabsorbent polymer having a vortex time of from 30 to 60 seconds and the particulate superabsorbent polymer has a particle diameter of larger than 600$\mu$m in an amount of from about 6wt% to about 15wt% of the particulate superabsorbent polymer as specified by standard sieve classification; or having a weight average particle diameter (D50) specified by standard sieve classification of from 350$\mu$m to about 500$\mu$m; or the particulate superabsorbent polymer having a particle diameters of smaller than 600$\mu$m and larger than 150$\mu$m in an amount of not less than about 85wt% of the particulate superabsorbent polymer composition and as specified by standard sieve classification; or having particles having a particle diameters of smaller than 600$\mu$m and larger than 150$\mu$m in an amount of not less than about 90wt% of the particulate superabsorbent polymer composition and as specified by standard sieve classification and the particles having a weight average particle diameter (D50) specified by standard sieve classification of from 300 to 400$\mu$m.

The present invention is also directed to a particulate superabsorbent polymer comprising having an internal crosslinking structure, from about 0.05 to about 2.0 wt.% of a foaming agent, and from about 0.001 to about 1.0 wt.% of a mixture of a lipophile nonionic surfactant and a polyethoxylated hydrophilic nonionic surfactant in an inside of the particle, the particle having a surface which has been subjected to a cross-linking treatment for cross-linking the surface, the particulate superabsorbent polymer having a vortex time of from 30 to 60 seconds and the particulate superabsorbent polymer further comprising a clay to form a superabsorbent polymer-clay hydrogel comprising from of about 90% to about 98%, by weight of superabsorbent polymer and from about 0.5% to about 10%, by weight of the clay; or comprising from about 0.001 to about 10 weight parts per 100 weight parts of the particulate superabsorbent polymer; or further comprising from about 0.05 to about 2.0 wt.% of a foaming agent added to the aqueous hydrogel.

The present invention is directed to a particulate superabsorbent polymer comprising having an internal crosslinking structure, from about 0.05 to about 2.0 wt.% of a foaming agent, and from about 0.001 to about 1.0 wt.% of a mixture of a lipophile nonionic surfactant and a polyethoxylated hydrophilic nonionic surfactant in an inside of the particle, the particle having a surface which has been subjected to a cross-linking treatment for cross-linking the surface, the particulate superabsorbent polymer having a vortex time of from 30 to 60 seconds and the particulate superabsorbent polymer further comprising a chelating agent wherein the chelating agent is selected from aminocarboxylic acids with at least three carboxyl groups and their salts.

The present invention is also directed to a particulate superabsorbent polymer comprising having an internal crosslinking structure, from about 0.05 to about 2.0 wt.% of a foaming agent, and from about 0.001 to about 1.0 wt.%

of a mixture of a lipophile nonionic surfactant and a polyethoxylated hydrophilic nonionic surfactant in an inside of the particle, the particle having a surface which has been subjected to a cross-linking treatment for cross-linking the surface, the particulate superabsorbent polymer having a vortex time of from 30 to 60 seconds and the particulate superabsorbent polymer further comprising from about 0.01 to 0.5 % weight of a thermoplastic polymer based on dry polymer powder weight; or wherein the thermoplastic polymer is selected from polyethylene, polyesters, polyurethanes, linear low density polyethylene (LLDPE), ethylene acrylic acid copolymer (EAA), styrene copolymers, ethylene alkyl methacrylate copolymer (EMA), polypropylene (PP), ethylene vinyl acetate copolymer (EVA) or blends thereof, or copolymers thereof; or wherein the thermoplastic polymer is added to the particulate superabsorbent polymer with the surface crosslinking agent.

The present invention is directed to a particulate superabsorbent polymer comprising having an internal crosslinking structure, from about 0.05 to about 2.0 wt.% of a foaming agent, and from about 0.001 to about 1.0 wt.% of a mixture of a lipophile nonionic surfactant and a polyethoxylated hydrophilic nonionic surfactant in an inside of the particle, the particle having a surface which has been subjected to a cross-linking treatment for cross-linking the surface, the particulate superabsorbent polymer having a vortex time of from 30 to 60 seconds and further comprising from 0.01wt% to about 5wt % based on the particulate superabsorbent polymer composition weight of a neutralized aluminum salt applied to the surface of the particulate superabsorbent polymer, in the form of an aqueous neutralized aluminum salt solution having a pH value from about 5.5 to about 8.

The present invention is also directed to a particulate superabsorbent polymer comprising having an internal crosslinking structure, from about 0.05 to about 2.0 wt.% of a foaming agent, and from about 0.001 to about 1.0 wt.% of a mixture of a lipophile nonionic surfactant and a polyethoxylated hydrophilic nonionic surfactant in an inside of the particle, the particle having a surface which has been subjected to a cross-linking treatment for cross-linking the surface, the particulate superabsorbent polymer having a vortex time of from 30 to 60 seconds and having a Centrifuge Retention Capacity (CRC) of from about 25grams to about 40grams of 0.9 weight percent sodium chloride aqueous per gram of the particulate superabsorbent polymer composition; or having a water content of from about 2 to about 10wt% of the particulate superabsorbent polymer; or having a Free Swell Gel Bed Permeability (FSGBP) of from about $20 \times 10^{-8}$ cm$^2$ to about $200 \times 10^{-8}$ cm$^2$; or having an 60-minute absorption capacity of from about 15g/g to about 26g/g under a load of 4.83 kPa for a 0.9weight %.

The present invention is also directed to a process for making a particulate superabsorbent polymer having fast water absorption comprising the steps of

a) preparing an aqueous monomer solution of a mixture of a of polymerizable unsaturated acid group containing monomer and an internal crosslinking agent monomer wherein the aqueous monomer solution comprises dissolved oxygen;
b) sparging the aqueous monomer solution of step a) including adding an inert gas to the aqueous monomer solution of step a) to replace the dissolved oxygen of the aqueous monomer solution;
c) polymerizing the aqueous monomer solution of step b) including the steps of

c1) adding to the aqueous monomer solution of step a): i) an aqueous solution comprising from about 0.05 to about 2.0 wt.% based on the total amount of the polymerizable unsaturated acid group containing monomer solution of a foaming agent; and ii) an aqueous solution comprising from about 0.001 to about 1.0 wt.% based on the total amount of the polymerizable unsaturated acid group containing monomer solution of a mixture of a lipophile nonionic surfactant and a polyethoxylated hydrophilic nonionic surfactant;
c2) treating the monomer solution of step c1) to high speed shear mixing of at least 2500 rpm to form a treated monomer solution, wherein the components i) an aqueous solution comprising from about 0.1 to about 1.0 wt.% of a foaming agent; and ii) an aqueous solution comprising from about 0.001 to about 1.0 wt.% of a mixture of a lipophile nonionic surfactant and a polyethoxylated hydrophilic nonionic surfactant are added to the aqueous monomer solution after step b) of sparging the aqueous monomer solution and before step c2) of high speed shear mixing of the aqueous monomer solution;
c3) forming a hydrogel by adding a polymerization initiator to the treated monomer solution of step c2) wherein the initiator is added to the treated monomer solution after the foaming agent and the surfactants, wherein the polymer is formed to include bubbles of the foaming agent into the polymer structure; and

d) drying and grinding the hydrogel of step c) to form particulate superabsorbent polymer; and
e) surface crosslinking the particulate superabsorbent polymer of step d) with a surface crosslinking agent wherein the surface crosslinked superabsorbent polymer has a vortex of from about 30 sec to about 60 sec.

The present invention further includes an absorbent article comprising a topsheet; a backsheet; and an absorbent core disposed between the topsheet and backsheet, the absorbent core comprising the foregoing particulate superabsorbent polymers of this invention.

A suitable superabsorbent polymer may be selected from synthetic, natural, biodegradable, and modified natural polymers. The term crosslinked used in reference to the superabsorbent polymer refers to any means for effectively rendering normally water-soluble materials substantially water-insoluble but swellable. Such a crosslinking means can include for example, physical entanglement, crystalline domains, covalent bonds, ionic complexes and associations, hydrophilic associations such as hydrogen bonding, hydrophobic associations or Van der Waals forces. A superabsorbent polymer as set forth in embodiments of the present invention may be obtained by the initial polymerization of from about 55% to about 99.9wt% of the superabsorbent polymer of polymerizable unsaturated acid group containing monomer. A suitable monomer includes any of those containing carboxyl groups, such as acrylic acid or methacrylic acid; or 2-acrylamido-2-methylpropanesulfonic acid, or mixtures thereof. It is desirable for at least about 50wt%, and more desirable for at least about 75wt% of the acid groups to be carboxyl groups.

The process to make a superabsorbent polymer of this invention may be obtained by the initial polymerization of from about 55% to about 99.9wt% of the superabsorbent polymer of polymerizable unsaturated acid group containing monomer. A suitable polymerizable monomer includes any of those containing carboxyl groups, such as acrylic acid, methacrylic acid, or 2-acrylamido-2-methylpropanesulfonic acid, or mixtures thereof. It is desirable for at least about 50% by weight, and more desirable for at least about 75wt% of the acid groups to be carboxyl groups.

The impurities contained in acrylic acid may be defined as follows. The acrylic acid may include protoanemonin and/or furfural, which may be controlled so as to be a predetermined amount or less in consideration of a color hue stability or a residual monomer. The content of the protoanemonin and/or furfural, may be from 0 to about 10 ppm, or from 0 to about 5 ppm, or from 0 to about 3 ppm, or from 0 to about 1 ppm. For the same reason, the acrylic acid may include a smaller amount (s) of aldehydes other than furfural and/or maleic acid. An amount(s) of aldehydes relative to acrylic acid is from 0 to about 5 ppm, or from 0 to about 3 ppm, or from 0 to about 1 ppm. Examples of aldehydes other than furfural encompass benzaldehyde, acraldehyde, acetaldehyde, and the like. From the view point of reducing a residual monomer, acrylic acid includes dimer acrylate in amount from 0 to about 500 ppm, or from 0 to about 200 ppm, or from 0 to about 100 ppm.

The acid groups are neutralized with an alkali base to the extent of at least about 25mol%, or from about 50mol% to about 80mol%, that is, the acid groups are desirably present as sodium, potassium, or ammonium salts. The amount of alkali base may be from about 14wt% to about 45wt% of the particulate superabsorbent polymer composition. The alkali base may include sodium hydroxide or potassium hydroxide. In some aspects, it is desirable to utilize polymers obtained by polymerization of acrylic acid or methacrylic acid, the carboxyl groups of which are neutralized in the presence of internal cross linking agents. It is noted that the neutralization may be achieved by either adding the alkali base to the monomer solution or adding the monomer such as acrylic acid to the alkali base. The temperature or neutralization (neutralization temperature) is not specifically limited, but may be from 10 to 100°C or from 30 to 90°C.

In some aspects, a second suitable monomer that can be copolymerized with the ethylenically unsaturated monomer may include, but is not limited to acrylamide, methacrylamide, hydroxyethyl acrylate, dimethylaminoalkyl (meth)-acrylate, ethoxylated (meth)-acrylates, dimethylaminopropylacrylamide, or acrylamidopropyltrimethylammonium chloride. Such monomer may be present in a range of from 0wt% to about 40wt% of the copolymerized monomer.

In the case when the monomer is acrylic acid, the partially neutralized, acrylate salt is turned into the polymer in the particulate water absorbing agent following polymerization, the converted value based on acrylic acid may be determined through converting the partially neutralized polyacrylate salt is assumed to be entirely the equimolar unneutralized polyacrylic acid.

The superabsorbent polymer of the invention also includes from about 0.001 wt% to about 5wt% by weight, or from about 0.2wt% to about 3wt% based on the total amount of the polymerizable unsaturated acid group containing monomer of at least one internal crosslinking agent. The internal crosslinking agent generally has at least two ethylenically unsaturated double bonds or one ethylenically unsaturated double bond and one functional group which is reactive towards acid groups of the polymerizable unsaturated acid group containing monomers or several functional groups which are reactive towards acid groups can be used as the internal crosslinking component and which is present during the polymerization of the polymerizable unsaturated acid group containing monomers. The internal crosslinker agent may contain a silane compound comprising at least one vinyl group or allyl group directly attached to a silicon atom and at least one Si-O bond.

Examples of internal crosslinking agents used in superabsorbent polymers include aliphatic unsaturated amides, such as methylenebisacryl- or -methacrylamide or ethylenebisacrylamide, and furthermore aliphatic esters of polyols or alkoxylated polyols with ethylenically unsaturated acids, such as di(meth)acrylates or tri(meth)acrylates of butanediol or ethylene glycol, polyglycols or trimethylolpropane, di- and triacrylate esters of trimethylolpropane which is preferably oxyalkylated, preferably ethoxylated, with 1 to 30 mol of alkylene oxide, acrylate and methacrylate esters of glycerol and pentaerythritol and of glycerol and pentaerythritol oxyethylated with from 1 to 30 mol of ethylene oxide and furthermore allyl compounds, such as allyl (meth)acrylate, alkoxylated allyl (meth)acrylate reacted with from 1 to 30 mol of ethylene oxide, triallyl cyanurate, triallyl isocyanurate, maleic acid diallyl ester, poly-allyl esters, vinyl trimethoxysilane, vinyl silane such as Dynasylan®6490, Dynasylan®6498, vinylalkoxysilanes such as vinyltrimethoxysilane, methylvinyltrimethoxysilane, vinyltriisoprppenoxysilane, vinyltriethoxysilane, methylvinyltriethoxysilane, vinylmethyldimethoxysilane, vinylethyldiethoxysilane, diethoxynethylvinylsilane, and vinyltris(2-methoxyethoxy)silane; vinylacetoxysilanes, such as vinylmethyldiacetoxysilane, vinylethyldiacetoxysilane and vinyltriacetoxysilane; allylalkoxysilanes such as allyltrimethoxysilane, allylmethyldimethoxysilane, and allyltriethoxysilane; divinylalkoxysilanes and divinylacetoxysilanes such as divinyldimethoxysilane, divinyldiethoxysilane and divinyldiacetoxysilane; diallylalkoxysilanes and diallylacetoxysilanes such as diallyldimethoxysilane, diallyldiethoxysilane and diallyldiacetoxysilane, vinyl triethoxysilane, polysiloxane comprising at least two vinyl groups, tetraallyloxyethane, tetraallyloxyethane, triallylamine, tetraallylethylenediamine, diols, polyols, hydroxy allyl or acrylate compounds and allyl esters of phosphoric acid or phosphorous acid, and furthermore monomers which are capable of crosslinking, such as N-methylol compounds of unsaturated amides, such as of methacrylamide or acrylamide, and the ethers derived there from. Ionic crosslinkers such as aluminum metal salts may also be employed, or other compounds containing mult-valent cations may also be employed. Post-polymerization reactive crosslinkers that either form additional crosslinks after polymerization, or those that hydrolyze or react to form fewer crosslinks after polymerization, for example, when triggered by temperature (during drying or thermal treatment for example), or by the addition of water or other chemical, or by a change in pH, or some other change, for example, when coming in contact with bodily fluids. Mixtures of the crosslinking agents mentioned can also be employed.

The superabsorbent polymer may include from about 0.001wt% to about 0.1wt% based on the total amount of the polymerizable unsaturated acid group containing monomer of a second internal crosslinker which may comprise compositions comprising at least two ethylenically unsaturated double-bonds, for example, methylenebisacrylamide or -methacrylamide or ethylenebisacrylamide; additionally, esters of unsaturated mono- or polycarboxylic acids of polyols, such as, diacrylates or triacrylates, e.g., butanediol- or ethylene glycol diacrylate or -methacrylate; trimethylolpropane triacrylate, as well as their alkoxylated derivatives; additionally, allyl compounds, such as allyl (meth)acrylate, triallyl cyanurate, maleic acid diallyl ester, polyallyl ester, tetraallyloxyethane, di- and triallylamine, tetrallylethylenediamine, allyl esters of phosphoric acid or phosphorous acid. Moreover, compounds having at least one functional group reactive towards acid groups may also be used. Examples thereof include N-methylol compounds of amides, such as methacrylamide or acrylamide, and the ethers derived there from, as well as di- and polyglycidyl compounds. The second internal crosslinker which may comprise polyethylene glycol monoallyl ether acrylate, ethoxylated trimethylol propane triacrylate, and/or polyethylene glycol diacrylate.

The usual initiators, such as e.g. azo or peroxo compounds, redox systems or UV initiators, (sensitizers), and/or radiation are used for initiation of the free-radical polymerization. In some aspects, initiators can be used for initiation of the free-radical polymerization. Suitable initiators include, but are not limited to, azo or peroxo compounds, redox systems or ultraviolet initiators, sensitizers, and/or radiation.

A polymerization initiator for use in the invention is selected as needed in accordance with the polymerization type, and not specifically limited. Examples of the polymerization initiator encompass a thermally degradable polymerization initiator, a photodegradable polymerization initiator, and a redox-type polymerization initiator, or the like. Specific examples of the thermally degradable polymerization initiator encompass persulfate such as sodium persulfate, potassium persulfate, or ammonium persulfate; and, peroxide such as hydrogen peroxide, t-butyl peroxide, or methyl ethyl ketone peroxide; and azo compound such as 2,2'-azobis(2-amidinopropane)dihydrochloride, 2.2'-azobis[2-(2-imidazolin-2-yl)propane]dihydrochloride, or the like. Specific examples of the photodegradable polymerization initiator encompass benzoin derivative, benzyl derivative, acetophenone derivative, benzophenone derivative, and azo compound, or the like. An example of the redox-type polymerization initiator encompasses use of a reducing compound such as L-ascorbic acid or sodium hydrogen sulfite in combination with persulfate or peroxide described above. A preferable example encompasses use of the photodegradable polymerization initiator in combination with the thermally degradable polymerization initiator described above. An using amount of the polymerization initiator may be from about 0.001 to about 1mole%, or from about 0.05 to 1.0mole% relative to the monomer. When the using amount of the polymerization initiator is more than 1% by mole, the coloration of the water absorbent resin may occur. Whereas, when the using amount of the polymerization initiator is less than 0.0001% by mole, it may cause an increase in a residual monomer.

Meanwhile, it should be noted that, instead of using the above-described polymerization initiator, the monomer may be polymerized by irradiation of activated energy rays such as radiation rays, electron beams, and UV rays. Further, the polymerization may be carried out by the use of the polymerization initiator in combination with the activated energy rays.

The foaming agent may include any alkali metal carbonate or alkali metal bicarbonate containing salt, or mixed salt, sodium carbonate, potassium carbonate, ammonium carbonate, magnesium carbonate, or magnesium (hydroxic) carbonates, calcium carbonate, barium carbonate, bicarbonates and hydrates of these, azo compounds or other cations, as well as naturally occurring carbonates, such as dolomite, or mixtures thereof. Foaming agents may include carbonate salts of multi-valent cations, such as Mg, Ca, Zn, and the like. Although certain of the multi-valent transition metal cations may be used, some of them, such as ferric cation, can cause color staining and may be subject to reduction-oxidation reactions or hydrolysis equilibria in water. This may lead to difficulties in quality control of the final polymeric product. Also, other multi-valent cations, such as Ni, Ba, Cd, Hg would be unacceptable because of potential toxic or skin sensitizing effects. The foaming agents may include sodium carbonate and sodium bicarbonate.

The present description further discloses an aqueous solution comprising from about 0.001 to about 1.0 wt.%, or from about 0.002 to about 0.5wt%, or from about 0.003 to about 0.1wt%, based on the total amount of the polymerizable unsaturated acid group containing monomer solution of a mixture of a lipophile surfactant and a polyethoxylated hydrophilic surfactant, wherein the lipophile surfactant may have a HLB of from 4 to 9 and the polyethoxylated hydrophilic surfactant has a HLB of from 12 to 18; or wherein the lipophile surfactant may be nonionic or the polyethoxylated hydrophilic surfactant may be nonionic.

Typical examples of the surfactant, polyoxy ethylene alkyl aryl ethers such as polyoxyethylene lauryl ether, polyoxyethylene cetyl ether, polyoxyethylene stearyl ether, polyoxyethylene oleyl ether, polyoxyethylene alkyl ethers like polyoxyethylene higher alcohol ethers, and polyoxyethylene nonyl phenyl ether; sorbitan fatty esters such as sorbitan monolaurate, sorbitan monopalmitate, sorbitan monostearate, sorbitan tristearate, sorbitan monooleate, sorbitan trioleate, sorbitan sesquioleate, and sorbitan distearate; polyoxyethylene sorbitan fatty esters such as polyoxyethylene sorbitan monolaurate, polyoxyethylene sorbitan monolaurate, polyoxyethylene sorbitan monopalmitate, polyoxyethylene sorbitan monostearate, polyoxy-ethylene sorbitan tristearate, polyoxyethylene sorbitan mono-oleate, and polyoxyethylene sorbitan trioleate; polyoxyethylene sorbitol fatty esters such as tetraoleic acid polyoxyethylene sorbit; glycerin fatty esters such as glycerol monostearate, glycerol monooleate, and self-eumlsifying glycerol monostearate; polyoxyethylene fatty esters such as polyethylene glycol mono-laurate, polyethylene glycol monostearate, polyethylene glycol distearate, and polyethylene glycol monooleate; polyoxyethylene alkyl amines; polyoxyethylene hardened castor oil; and alkyl alcohol amines may be cited. The mixture of nonionic surfactant may include a mixture of lipophile surfactant is a sorbitan ester and the polyethoxylated hydrophilic surfactant is a polyethoxylated sorbitan ester.

The method of this invention is to perform the polymerization or copolymerization reaction in the presence of a mixture of mixture of a lipophile surfactant and a polyethoxylated hydrophilic surfactant. The use of the mixture of two surfactants enables the bubbles to be stably dispersed. Further by appropriately controlling the kind and the amount of the mixture of two surfactants, the pore diameter and the water-absorbent speed of the hydrophilic polymer to be produced can be controlled. A process to make the particulate superabsorbent polymer having fast water absorption of the present invention includes the steps of:

a) preparing a monomer solution comprising

a1) from about 55 to about 99.9 wt.% of polymerizable unsaturated acid group containing monomers;
a2) from about 0.001 to about 5.0 wt.% of an internal crosslinking agent;
a3) from about 14 to 45 wt% of an alkali wherein the composition has a degree of neutralization of from about 50 mol % to about 70 mol %; and
a4) sparging the monomer solution of step c) by bubbling an inert gas into the monomer solution so that the monomer solution has less than 1wt % of oxygen;

b) polymerizing the monomer solution of step a) including the steps of

b1) adding to the monomer solution of step a) the following:

i) an aqueous solution comprising from about 0.05 to about 2.0 wt.% based on the total amount of the polymerizable unsaturated acid group containing monomer solution of a foaming agent; and
ii) an aqueous solution comprising from about 0.001 to about 1.0 wt.% based on the total amount of the polymerizable unsaturated acid group containing monomer solution of a mixture of a lipophile surfactant and a polyethoxylated hydrophilic surfactant;

b2) treating the monomer solution of step a) and step b1) to high speed shear mixing which may be at least about 2500 rpm to form a treated monomer solution;
b3) forming a hydrogel by adding a polymerization initiator to the treated monomer solution of step b2) wherein the initiator is added to the treated monomer solution after the foaming agent and the surfactants, wherein the polymer is formed to include bubbles of the foaming agent into the polymer structure and wherein the bubbles; and

c) drying and grinding the hydrogel of step b) to form particulate superabsorbent polymer; and
d) surface crosslinking the particulate superabsorbent polymer of step c) with a surface crosslinking agent wherein the surface crosslinked superabsorbent polymer has a vortex of from about 30 sec to about 60 sec.

The temperature at the start of the polymerization, though variable with the kind of the radical polymerization initiator to be used, maybe in the range of 0-50°C, or in the range of 10°C-40°C. The polymerization temperature in the process of reaction, though variable with the kind of the radical polymerization initiator to be used, maybe in the range of 20°C-110°C, or in the range of 30°C-90°C. If the temperature at the start of the polymerization or the polymerization temperature in the process of reaction deviates from the range mentioned above, such disadvantages as (1) an undue increase in the amount of the residual monomer in the produced water-absorbent resin, (b) difficulty incurred in the control of the foaming with a foaming agent which will be described specifically herein below, and (c) excessive advance of the self-crosslinking reaction accompanied by an undue decrease in the amount of water absorbed by the water-absorbent resin will possibly ensue.

The reaction time is not particularly limited but is only required to be set depending on the combination of an unsaturated monomer, a cross-linking agent, and a radical polymerization initiator or on such reaction conditions as the reaction temperature.

The average pore diameter of the water-absorbent resin or the hydrophilic polymer which has almost independent bubble structure is in the range of 10-500$\mu$m, or in the range of 20-400$\mu$m, or in the range of 30-300$\mu$m, or in the range of 40-200$\mu$m, or in the range of 70$\mu$m to about 110$\mu$m. The pore diameter mentioned above is found by subjecting the cross section of the water-absorbent resin or the hydrophilic polymer in a dry state to image analysis with the aid of an electron microscope. Specifically, the average pore diameter is obtained by forming a histogram representing the distribution of pore diameters of the water-absorbent resin in consequence of the image analysis and calculating the number average of pore diameters based on the histogram.

To further enhance the properties of the particulate superabsorbent polymer, an additional amount of foaming agent as set forth herein may also be added to the aqueous superabsorbent polymer prior to the drying step.

The drying temperature is not particularly limited but is required to fall in the range of 100°C-250°C, or in the range of 120°C-200°C, for example. Though the drying time is not particularly limited, it is preferred to be in the approximate range of 10 seconds-5 hours. The hydrogel resin may be neutralized or further disintegrated for fine division prior to the drying.

The drying method to be adopted is not particularly limited but may be selected from among various methods such as, for example, drying by heating, drying with hot air, drying under a reduced pressure, drying with an infrared ray, drying with a microwave, drying in a drum drier, dehydration by azeotropy with a hydrophobic organic solvent, and a high-humidity drying by the use of hot steam. Among the methods of drying mentioned above, the drying with hot air and the drying with a microwave prove particularly favorable. When the hydrogel containing the bubbles is irradiated with a microwave, the produced water-absorbent resin enjoys a further exalted water absorption speed because the bubbles are consequently expanded to several to some tens of times the original volume.

Particulate Superabsorbent Polymer (SAP) - Clay Blend

As previously noted, the polymerization reaction proceeds rapidly to yield a highly viscous hydrogel that is extruded, for example, onto a flat surface such as a continuously moving conveyor belt. The neutralized superabsorbent polymer hydrogel then is comminuted, and the clay may be added to, typically as aqueous clay slurry, and intimately admixed with, the comminuted superabsorbent polymer hydrogel particles. The clay may also be added as solid particles, or a powder. The SAP hydrogel and clay components may then be intimately admixed, e.g., by extrusion, to disperse the clay in and on the hydrogel particles. The resulting neutralized SAP-clay mixture is then dried and sized, and optionally surface crosslinked to provide neutralized SAP-clay particles. Comminution of the SAP-clay hydrogel particles may be performed simultaneously or sequentially.

After comminutation, the viscous SAP-clay hydrogel particles are dehydrated (i.e., dried) to obtain SAP-clay particles in a solid or powder form. The dehydration step may be performed, for example, by heating the viscous SAP-clay hydrogel particles at a temperature of from about 190°C to about 210°C for about 15 minutes to about 120 minutes in a forced-air oven, or a time period of from about 15 minutes to about 110 minutes or from about 15 minutes to about 100 minutes, or from about 20 minutes to about 100 minutes. The dried SAP-clay hydrogel may then be subjected to further mechanical means for particle size reduction and classification including chopping, grinding, and sieving.

Such SAP-clay compositions may include superabsorbent polymer present in an amount of about 90% to about 99.5%, or from about 91% to about 99%, or from about 92% to about 98% by weight, and the clay is present in an amount of about 0.5% to about 10%, or from about 1 to about 9wt%, or from about 2 to about 8wt% by weight.

Clay useful in the present SAP-clay particles can be swelling or nonswelling clay. Swelling clays have the ability to absorb water and are swellable, layered organic materials. Suitable swelling clays include, but are not limited to, montmorillonite, saponite, nontronite, laponite, beidelite, hectorite, sauconite, stevensite, vermiculite, volkonskoite, magadite, medmontite, kenyaite, and mixtures thereof.

Suitable nonswelling clays include, without limitation, kaolin minerals (including kaolinite, dickite, and nacrite), serpentine minerals, mica minerals (including illite), chlorite minerals, sepolite, palygorskite, bauxite, and mixtures thereof.

The clay also can be an organophilic clay. As used here and hereafter, the term "organophilic" is defined as the property of a compound to absorb at least its own weight, and preferably many times its own weight, of an organic, water-immiscible compound. An organophilic compound optionally can absorb water or a water-miscible compound.

Commercially available clays include ULTRAGLOSS® clays (hydrous kaolin) from BASF Corporation, Florham Park, N.J.; Purified Clay from Nanocor Technologies, Arlington Heights, Ill.; and HYDROGLOSS® from Huber, Atlanta, Ga.

Particle Size

The polymerization forms a superabsorbent polymer gel, which is granulated into superabsorbent polymer particles, or particulate superabsorbent polymer. The superabsorbent polymer gel generally has moisture content of from about 40 to 80wt% of the superabsorbent polymer gel. The particulate superabsorbent polymer generally includes particle sizes ranging from about 50$\mu$m to about 1000$\mu$m, or from about 150$\mu$m to about 850$\mu$m. The present invention may include at least about 40wt% of the superabsorbent polymer particles having a particle size from about 300$\mu$m to about 600$\mu$m, at least about 50wt% of the particles having a particle size from about 300$\mu$m to about 600$\mu$m, or at least about 60wt% of the particles having a particle size from about 300$\mu$m to about 600$\mu$m as measured by screening through a U.S. standard 30 mesh screen and retained on a U.S. standard 50 mesh screen. In addition, the mass average particle diameter D50 may be from 200 to 450$\mu$m, or from 300 to 430$\mu$m.

Further, the percentage of particles less than 150$\mu$m is generally 0-8% by mass, or 0-5% by mass, or 0-3% by mass, or 0-1% by mass. Further, the percentage of particles more than 600$\mu$m may be from 0 to 25% by mass, or from 3 to 15% by mass, or from 5 to 12% by mass, or 5 to 8% by mass, as measured using for example a RO-TAP® Mechanical Sieve Shaker Model B available from W. S. Tyler, Inc., Mentor Ohio.

The particle size may be adjusted by subjecting the particles to dispersion polymerization and dispersion drying. However, in general, when carrying out aqueous polymerization in particular, the particles are pulverized and classified after drying, and then mass average diameter of D50, and the amount of particles smaller than 150$\mu$m and larger than 600$\mu$m, is adjusted so as to obtain a specific particle size distribution. For example, if the specific particle size distribution is achieved by decreasing the diameter of the particles having mass average diameter of D50 to 400$\mu$m or smaller and also reducing the amount of the fine particles having diameter less than 150$\mu$m and larger than 600$\mu$m, the particles may be first classified into coarse particles and fine particles after drying by using a general classifying equipment such as a sieve. This process preferably removes coarse particles with a diameter of 5000$\mu$m to 600$\mu$m, or of 2000$\mu$m to 600$\mu$m, or of 1 000$\mu$m to 600$\mu$m. Then, in the main adjustment process, the fine particles with a diameter less than 150$\mu$m, are removed. The removed coarse particles may be discarded, but they are more likely to be pulverized again through the foregoing pulverizing process. The resulting particulate superabsorbent polymer thus produced with a specific particle size distribution through the pulverizing process is therefore constituted of irregularly-pulverized particles.

The particulate superabsorbent polymers are surface treated with additional chemicals and treatments as set forth herein. In particular, the surface of the particulate superabsorbent polymer may be crosslinked, generally referred to as surface crosslinking, by the addition of a surface crosslinking agent and heat-treatment. In general, surface crosslinking is a process to increase the crosslink density of the polymer matrix in the vicinity of the particulate superabsorbent polymer surface with respect to the crosslinking density of the particle interior. The amount of the surface crosslinking agent may be present in an amount of from about 0.01wt% to about 5wt% of the dry particulate superabsorbent polymer composition, and such as from about 0.1 wt% to about 3wt%, and such as from about 0.1wt% to about 1wt% by weight, based on the weight of the dry particulate superabsorbent polymer composition.

Desirable surface crosslinking agents include chemicals with one or more functional groups that are reactive toward pendant groups of the polymer chains, typically the acid groups. Surface crosslinker agents comprise functional groups which react with functional groups of a polymer structure in a condensation reaction (condensation crosslinker), in an addition reaction or in a ring opening reaction. These compounds may include, for example, diethylene glycol, triethylene glycol, polyethylene glycol, glycerine, polyglycerine, propylene glycol, diethanolamine, triethanolamine, polyoxypropylene, oxyethylene-oxypropylene block copolymers, sorbitan fatty acid esters, polyoxyethylene sorbitan fatty acid esters, trimethylolpropane, pentaerythritol, polyvinyl alcohol, sorbitol, 1,3-dioxolan-2-one (ethylene carbonate), 4-methyl-1,3-dioxolan-2-one (propylene carbonate), or 4,5-dimethyl-1,3 -dioxolan-2-one.

After the particulate superabsorbent polymer has been brought into contact with the surface crosslinker agent, or with the fluid comprising the surface crosslinker agent, the treated particulate superabsorbent polymer is heat treated to a temperature of from about 50 to about 300°C, or from about 75 to about 275°C, or from about 150 to about 250°C, and for a time of from about 5 to about 90 minutes dependent on the temperature, so that the outer region of the polymer structures is more strongly crosslinked compared to the inner region (i.e., surface crosslinking). The duration of the heat treatment is limited by the risk that the desired property profile of the polymer structures will be destroyed as a result of the effect of heat.

In one particular aspect of surface crosslinking, the particulate superabsorbent polymer is surface-treated with ethylene carbonate followed by heating to affect surface crosslinking of the superabsorbent polymer particle, which improves the surface crosslinking density and the gel strength characteristics of the particulate superabsorbent polymer.

More specifically, the surface crosslinking agent is coated onto the particulate superabsorbent polymer by mixing the particulate superabsorbent polymer with an aqueous alcoholic solution of the ethylene carbonate surface crosslinking agent. The amount of alcohol in the aqueous alcoholic solution may be determined by the solubility of the alkylene carbonate and is kept as low as possible for various reasons, for instance, for protection against explosions. Suitable alcohols are methanol, isopropanol, ethanol, butanol, or butyl glycol, as well as mixtures of these alcohols. In some aspects, the solvent desirably is water, which typically is used in an amount of about 0.3% by weight to about 5.0wt%, based on the weight of the dry particulate superabsorbent polymer composition. In still other aspects, the ethylene carbonate surface crosslinking agent may be applied from a powder mixture, for example, with an inorganic carrier material, such as silicone dioxide ($SiO_2$), or in a vapor state by sublimation of the ethylene carbonate.

To achieve the desired surface crosslinking properties, the surface crosslinking agents such as ethylene carbonate should be distributed evenly on the particulate superabsorbent polymer. For this purpose, mixing is effected in suitable mixers known in the art, such as fluidized bed mixers, paddle mixers, rotary drum mixers, or twin-worm mixers. It is also possible to carry out the coating of the particulate superabsorbent polymer during one of the process steps in the production of the particulate superabsorbent polymer. In one particular aspect, a suitable process for this purpose is the inverse suspension polymerization process.

The solution of the surface crosslinking agent may also include a from 0wt% to about 1wt%, or from about 0.01wt% to about 0.5wt% based on the dry particulate superabsorbent polymer composition of a thermoplastic polymer. Examples of thermoplastic polymers include polyolefin, polyethylene, polyester, linear low density polyethylene (LLDPE), ethylene acrylic acid copolymer (EAA), ethylene alkyl methacrylate copolymer (EMA), polypropylene (PP), maleated polypropylene, ethylene vinyl acetate copolymer (EVA), polyester, and blends of all families of polyolefins, such as blends of PP, EVA, EMA, EEA, EBA, HDPE, MDPE, LDPE, LLDPE, and/or VLDPE, may also be advantageously employed. In particular aspects, maleated polypropylene is a preferred thermoplastic polymer for use in the present invention. A thermoplastic polymer may be functionalized to have additional benefits such as water solubility or dispersability.

The heat treatment, which follows the coating treatment of the particulate superabsorbent polymer, may be carried out as follows. In general, the heat treatment is at a temperature of from about 100°C to about 300°C. Lower temperatures are possible if highly reactive epoxide crosslinking agents are used. However, if an ethylene carbonate is used, then the thermal treatment is suitably at a temperature of from about 150°C to about 250°C. In this particular aspect, the treatment temperature depends on the dwell time and the kind of ethylene carbonate. For example, at a temperature of about 150°C, the thermal treatment is carried out for one hour or longer. In contrast, at a temperature of about 250°C, a few minutes (e.g., from about 0.5 minutes to about 5 minutes) are sufficient to achieve the desired surface crosslinking properties. The thermal treatment may be carried out in conventional dryers or ovens known in the art.

The particulate superabsorbent polymer compositions may be further surface treated before, during or after surface crosslinking with other chemical compositions.

The particulate superabsorbent polymer composition according to the invention may comprise from about 0.01wt% to about 5wt % based on the particulate superabsorbent composition weight of an aluminum salt applied to the surface of the particulate superabsorbent polymer, in the form of an aqueous solution having a pH value from about 5.5 to about 8, or from about 6 to about 7. Or, the particulate superabsorbent polymer composition comprises from about 6wt% to about 15wt % based on the particulate superabsorbent composition weight of an aqueous aluminum salt solution applied to the surface of the surface crosslinked particulate superabsorbent polymer, wherein the aqueous aluminum salt solution has a pH value from about 5.5 to about 8, or from about 6 to about 7. The aqueous solution of the aluminum salt may comprise an aluminum cation and a hydroxyl ion or an anion of a deprotonated hydroxyl organic acid. Examples of preferred organic acids are hydroxyl monocarboxylic acids such as lactic acid, glycolic acid, gluconic acid, or 3-hydroxypropionic acid.

The aqueous aluminum salt solution includes the reaction product of alkali hydroxide and aluminum sulfate or aluminum sulfate hydrate. In another embodiment, the aqueous aluminum salt solution includes the reaction product of sodium hydroxide and aluminum sulfate or aluminum sulfate hydrate. In yet another embodiment, the aqueous aluminum salt solution comprises an aluminum compound and an organic acid. The mixture of the aluminum compound with the organic acid (salt) can be acidic or basic. And the pH can be adjusted to the desired range with a basic or acidic material. Examples of the basic materials for pH adjustment include but not limited to sodium hydroxide, potassium hydroxide, ammonium hydroxide, sodium carbonate or sodium bicarbonate. Examples of the acidic materials for pH adjustment include but are not limited to hydrochloric acid, sulfuric acid, methylsulfonic acid, or carbon dioxide in water. The acidic aluminum salts, such as aluminum chloride, aluminum sulfate, aluminum nitrate and polyaluminum chloride, or the basic aluminum salts, such as sodium aluminate, potassium aluminate and ammonium aluminate, may be used for pH adjustment as well.

The aqueous aluminum salt solution may be added at various stages of surface treatment of the particulate superabsorbent polymer. In one embodiment, the aqueous aluminum salt solution may be applied to the particulate superabsorbent polymer along with the surface crosslinking solution.

The aqueous aluminum salt solution may be added after the surface crosslinking step, which may be called a post treatment. In one embodiment, the surface crosslinked particulate superabsorbent polymer and the aluminum salt are mixed using means well known to those skilled in the art. In particular, from about 6wt% to about 15wt% of an aqueous aluminum salt solution is applied to a surface crosslinked particulate superabsorbent polymer composition.

The particulate superabsorbent polymer compositions according to the invention may be surface treated, before, during or after surface crosslinking with from 0.01wt% to about 5wt % based on the particulate superabsorbent composition weight of a compound containg one or more multivalent metal cations applied to the surface of the particulate superabsorbent polymer. Examples include cations of aluminum, calcium, iron, zinc, magnesium and zirconium. Of these, aluminum may be used. Examples of anions in the multivalent metal salt may include halides, chlorohydrates, sulfates, nitrates and acetates. Aluminum sulfate may be used and is readily commercially available. The aluminum sulfate may be hydrated aluminum sulfate, wherein aluminum sulfate may have from 12 to 14 waters of hydration. Mixtures of multivalent metal salts can be employed. In one embodiment, the invention may comprise from about 0.01wt% to about 5wt % based on the particulate superabsorbent composition weight of a aluminum salt applied to the surface of the particulate superabsorbent polymer, in the form of an aqueous solution having a pH value from about 5.5 to about 8, or from about 6 to about 7.

Aqueous aluminum salt solution may include the reaction product of alkali hydroxide and aluminum sulfate or aluminum sulfate hydrate. In another embodiment, the aqueous aluminum salt solution comprises a single aluminum salt such as aluminum sulfate or aluminum chloride, or mixtures with other multivalent cation-containing compounts that may be applied with or without pH adjustment. In yet another embodiment, the aqueous aluminum salt solution may comprise an aluminum compound and an organic acid. The mixture of the aluminum compound with the organic acid (salt) can be acidic or basic and may be applied with or without pH adjustment. If a pH adjustment is desired, the pH can be adjusted to the desired range with a basic or acidic material.

The particulate superabsorbent polymer compositions according to the invention may be surface treated with from about 0.01% to about 2% by weight, or from about 0.01% to about 1% by weight based on the dry superabsorbent polymer composition of a *water-insoluble* inorganic metal compound. The water-insoluble inorganic metal compound may include a cation selected from aluminum, titanium, calcium, or iron and an anion selected from phosphate, borate, or chromate. An example of a water insoluble inorganic metal compound includes aluminum phosphate. The inorganic metal compound may have a mass median particle size of less than about $2\mu m$ and may have a mass median particle size of less than about $1\mu m$.

The particulate superabsorbent polymer composition may include from about 0wt% to about 5wt%, or from about 0.001wt% to about 3wt%, or from about 0.01wt% to about 2wt% based on the weight of the dry particulate superabsorbent polymer composition of a cationic polymer. A cationic polymer as used herein refers to a polymer or mixture of polymers comprising a functional group or groups having a potential of becoming positively charged ions upon ionization in an aqueous solution. Suitable functional groups for a cationic polymer include, but are not limited to, primary, secondary, or tertiary amino groups, imino groups, imido groups, amido groups, and quaternary ammonium groups. Examples of synthetic cationic polymers include the salts or partial salts of poly(vinyl amines), poly(allylamines), or poly(ethylene imine). Examples of natural-based cationic polymers include partially deacetylated chitin, chitosan, and chitosan salts.

The particulate superabsorbent polymer composition may include from about 0wt% to about 5wt%, or from about 0.001wt% to about 3wt%, or from about 0.01wt% to about 2wt% based on the weight of the dry particulate superabsorbent polymer composition of water-insoluble, inorganic powder. Examples of insoluble, inorganic powders include silicon dioxide, silica, titanium dioxide, aluminum oxide, magnesium oxide, zinc oxide, talc, calcium phosphate, clays, diatomaceous earth, zeolites, bentonite, kaolin, hydrotalcite, activated clays, etc. The insoluble inorganic powder additive may be a single compound or a mixture of compounds selected from the above list. Examples of silica include fumed silica, precipitated silica, silicon dioxide, silicic acid, and silicates. In some particular aspects, microscopic noncrystalline silicon dioxide is desirable. Products include SIPERNAT® 22S and AEROSIL® 200 available from Evonik Corporation, Parsippany, New Jersey. In some aspects, the particle diameter of the inorganic powder can be $1,000\mu m$ or smaller, such as $100\mu m$ or smaller.

The particulate superabsorbent polymer composition may also include from 0wt% to about 30wt%, or from about 0.001wt% to about 25wt%, or from about 0.01wt% to about 20wt% based on the weight of the dry particulate superabsorbent polymer composition, of water-soluble polymers, such as partly or completely hydrolyzed polyvinyl acetate, polyvinylpyrrolidone, starch or starch derivatives, polyglycols or polyacrylic acids, preferably in polymerized-in form. The molecular weight of these polymers is not critical as long as they are water-soluble. Preferred water-soluble polymers are starch and polyvinyl alcohol. The content of such water-soluble polymers in the absorbent polymer according to the invention is 0-30wt%, or 0-5wt%, based on the total amount of the dry particulate superabsorbent polymer composition. The water-soluble polymers, preferably synthetic polymers, such as polyvinyl alcohol, can also serve as a graft base for the monomers to be polymerized.

The particulate superabsorbent polymer composition may also include from 0wt% to about 5 wt %, or from about 0.001wt% to about 3wt%, or from about 0.01wt% to about 2 wt% based on the weight of the dry particulate superabsorbent polymer composition, of dedusting agents, such as hydrophilic and hydrophobic dedusting agents such as those described in U.S. Patents 6,090,875 and 5,994,440.

In some aspects, additional surface additives may optionally be employed with the particulate superabsorbent polymer composition, such as odor-binding substances, such as cyclodextrins, zeolites, inorganic or organic salts, and similar materials; anti-caking additives, flow modification agents, surfactants, viscosity modifiers, and the like.

The particulate superabsorbent polymer composition of the present invention may be, after the heat treatment step, treated with an aqueous solution, such as the aqueous solution of deprotonated organic acid salt, aluminum salt, or water soluble polymer such as polyethylene glycol.

The treated particulate superabsorbent polymer composition has moisture content of from about 3wt% to about 15wt%, or from about 4wt% to about 12wt%, or from 5wt% to about 11wt% based on the particulate superabsorbent polymer composition and as measured by the Moisture Content Test contained herein.

In still another embodiment, a chelating agent may be added to and become part of the particulate superabsorbent polymer composition of the present invention. The chelating agent may be selected from specific amino carboxylic acids can be fixed onto the surface of the water-absorbent resin by mixing the amino polycarboxylic acid and the surface-crosslinking agent with a water-absorbent resin before surface-crosslinking the water-absorbent resin, thus surface-crosslinking the water-absorbent resin, or by adding the amino polycarboxylic acid and water to with a specific surface-crosslinked water-absorbent resin, thus granulating this resin. Because the deterioration of water-absorbent resins occurs from their surfaces, it is preferable that the chelating agent is put in the neighborhood of the surface of the particulate superabsorbent polymer composition. In one of the present invention production processes, for example, the chelating agent and the surface-crosslinking agent, which is reactable upon a carboxyl group, are mixed with the above-obtained superabsorbent polymer having a carboxyl group.

Examples of the chelating agent, as used in the present invention, include the following compounds: (1) aminocarboxylic acids and their salts; (2) monoalkylcitramides, monoalkenylcitramides, and their salts; (3) monoalkylmalonamides, monoalkenylmalonamides, and their salts; (4) monoalkylphosphoric esters, monoalkenylphosphoric esters, and their salts; (5) N-acylated glutamic acids, N-acylated aspartic acids, and their salts; (6) .beta.-diketone derivatives; (7) tropolone derivatives; and (8) organic phosphoric acid compounds.

The amount of the chelating agent is generally from about 0.0001 to about 10 weight parts, or from about 0.0002 to about 5 weight parts, per 100 weight parts of the solid content of the particulate superabsorbent polymer composition. In the present invention, the chelating agent may be added to the particulate superabsorbent polymer either during surface crosslinking or to a surface crosslinked particulate superabsorbent polymer.

The particulate superabsorbent polymer composition of the present invention exhibits certain characteristics, or properties, as measured by Free Swell Gel Bed Permeability (FSGBP), Centrifuge Retention Capacity (CRC), and absorbency under load at about 0.9psi (0.9 psi AUL). The FSGBP Test is a measurement of the permeability of a swollen bed of particulate superabsorbent polymer composition in terms of $10^{-8}cm^2$ (e.g., separate from the absorbent structure) under a confining pressure after what is commonly referred to as "free swell" conditions. In this context, the term "free swell" means that the particulate superabsorbent polymer composition is allowed to swell without a swell restraining load upon absorbing test solution as will be described.

Permeability is a measure of the effective connectedness of a porous structure, be it a mat of fiber or a slab of foam or, in the case of this application, particulate superabsorbent polymer and particulate superabsorbent polymer composition, generally referred to as particulate superabsorbent polymer compositions herein, or SAP, and may be specified in terms of the void fraction and extent of connectedness of the particulate superabsorbent polymer compositions. Gel permeability is a property of the mass of particulate superabsorbent polymer compositions as a whole and is related to particle size distribution, particle shape, the connectedness of the open pores, shear modulus and surface modification of the swollen gel. In practical terms, the permeability of the particulate superabsorbent polymer composition is a measure of how rapidly liquid flows through the mass of swollen particles. Low permeability indicates that liquid cannot flow readily through the particulate superabsorbent polymer compositions, which is generally referred to as gel blocking, and that any forced flow of liquid (such as a second application of urine during use of the diaper) must take an alternate path (e.g., diaper leakage).

The Vortex Test measures the amount of time in seconds required for 2 grams of a SAP to close a vortex created by stirring 50 milliliters of saline solution at 600 revolutions per minute on a magnetic stir plate. The time it takes for the vortex to close is an indication of the free swell absorbing rate of the SAP.

The Centrifuge Retention Capacity (CRC) Test measures the ability of the particulate superabsorbent polymer composition to retain liquid therein after being saturated and subjected to centrifugation under controlled conditions. The resultant retention capacity is stated as grams of liquid retained per gram weight of the sample (g/g).

The Absorbency Under Load (AUL) Test measures the ability of the particulate superabsorbent polymer composition particles to absorb a 0.9 weight percent solution of sodium chloride in distilled water at room temperature (test solution) while the material is under a load of 0.9psi.

The Pressure Absorbency Index (PAI) is the sum of the Absorbency Under Load values (described herein below) for a SAP determined under the following loads: 0.01 pound per square inch (690 dynes per square centimeter); 0.29 pound per square inch (19995 dynes per square centimeter); 0.57 pound per square inch (39300 dynes per square centimeter); and 0.90 pound per square inch (62053 dynes per square centimeter). That is, the Absorbency Under Load values for a given SAP are determined under the restraining forces set forth above according to the method set forth below in connection with the examples. The Absorbency Under Load values determined under the restraining loads set forth above are then totaled to produce the Pressure Absorbency Index.

All values of Centrifuge Retention Capacity, Absorbency Under Load and Gel Bed Permeability set forth herein are to be understood as being determined by the Centrifuge Retention Capacity Test, Absorbency Under Load Test, and Free Swell Gel Bed Permeability Test as provided herein.

The particulate superabsorbent polymer composition having fast absorption made by a process of the present invention may have vortex time of from about 30 to 60 sec, or from 40 sec to about 60 sec, a centrifuge retention capacity of from about 25g/g to about 40g/g, or from about 27 to about 35g/g; and an absorbency under load at 0.9psi of from about 15g/g to about 24g/g, or from about 16g/g to about 22g/g, a PAI of from about 120 to about 140, and an original Free Swell Gel Bed Permeability (FSGBP) of about $20 \times 10^{-8}$ cm$^2$ to about $200 \times 10^{-8}$ cm$^2$.

The particulate superabsorbent polymer compositions according to the present invention can be employed in many absorbent articles including sanitary towels, diapers, or wound coverings, and they have the property that they rapidly absorb large amounts of menstrual blood, urine, or other body fluids. Since the agents according to the invention retain the absorbed liquids even under pressure and are also capable of distributing further liquid within the construction in the swollen state, they are more desirably employed in higher concentrations, with respect to the hydrophilic fiber material, such as fluff, when compared to conventional current superabsorbent compositions. They are also suitable for use as a homogeneous superabsorber layer without fluff content within the diaper construction, as a result of which particularly thin articles are possible. The polymers are furthermore suitable for use in hygiene articles (e.g., incontinence products) for adults.

The particulate superabsorbent polymer compositions according to the invention are also employed in absorbent articles that are suitable for further uses. In particular, the particulate superabsorbent polymer compositions of this invention can be used in absorbent compositions for absorbents for water or aqueous liquids, or in constructions for absorption of body fluids, in foamed and non-foamed sheet-like structures, in packaging materials, in constructions for plant growing, as soil improvement agents or as active compound carriers. For this, they are processed to a web by mixing with paper or fluff or synthetic fibers or by distributing the superabsorbent polymers between substrates of paper, fluff or non-woven textiles or by processing into carrier materials.

The particulate superabsorbent polymer compositions according to the present invention can be employed in many absorbent articles including sanitary towels, diapers, or wound coverings, and they have the property that they rapidly absorb large amounts of menstrual blood, urine, or other body fluids. Since the agents according to the invention retain the absorbed liquids even under pressure and are also capable of distributing further liquid within the construction in the swollen state, they are more desirably employed in higher concentrations, with respect to the hydrophilic fiber material, such as fluff, when compared to conventional current superabsorbent compositions. They are also suitable for use as a homogeneous superabsorber layer without fluff content within the diaper construction, as a result of which particularly thin articles are possible. The polymers are furthermore suitable for use in hygiene articles (e.g., incontinence products) for adults.

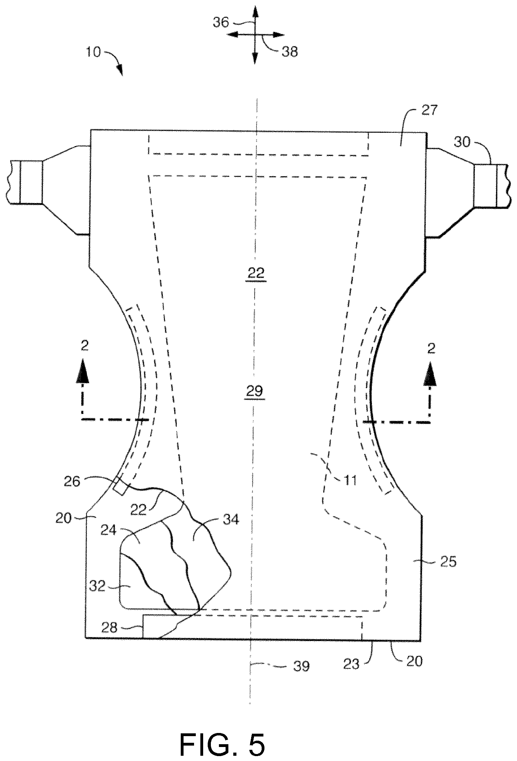

For example, referring now to FIG 5, in one aspect, the absorbent article employing the particulate superabsorbent polymer composition described herein is a disposable article 10 including a backsheet or outer cover 20, a liquid permeable topsheet or bodyside liner 22 positioned in facing relation with the outer cover 20, and an absorbent core 24, such as an absorbent pad, that is located between the bodyside liner 22 and the outer cover 20. The article 10 has an outer surface 23, a front waist region 25, a back waist region 27, and a crotch region 29 connecting the front and back waist regions 25, 27. The outer cover 20 defines a length and a width that, in the illustrated aspect, coincide with the length and width of the article 10. The absorbent core 24 generally defines a length and width that are less than the length and width of the outer cover 20, respectively. Thus, marginal portions of the article 10, such as marginal sections of the outer cover 20, can extend past the terminal edges of the absorbent core 24. In the illustrated aspects, for example, the outer cover 20 extends outwardly beyond the terminal marginal edges of the absorbent core 24 to form side margins and end margins of the article 10. The bodyside liner 22 is generally coextensive with the outer cover 20 but can optionally cover an area that is larger or smaller than the area of the outer cover 20, as desired. In other words, the bodyside liner 22 is connected in superposed relation to the outer cover 20. The outer cover 20 and bodyside liner 22 are intended to face the garment and body of the wearer, respectively, while in use.

To provide improved fit and to help reduce leakage of body exudates from the article 10, the article side margins and end margins can be elasticized with suitable elastic members, such as single or multiple strands of elastic. The elastic strands can be composed of natural or synthetic rubber and can optionally be heat shrinkable or heat elasticizable. For example, as representatively illustrated in FIG 5, the article 10 can include leg elastics 26 that are constructed to operably gather and shirr the side margins of the article 10 to provide elasticized leg bands that can closely fit around the legs of the wearer to reduce leakage and provide improved comfort and appearance. Similarly, waist elastics 28 can be employed to elasticize the end margins of the article 10 to provide elasticized waists. The waist elastics 28 are configured to operably gather and shirr the waist sections to provide a resilient comfortably close fit around the waist of the wearer. In the illustrated aspects, the elastic members are illustrated in their uncontracted, stretched condition for the purpose of clarity.

Fastening means, such as hook and loop fasteners 30, can be employed to secure the article 10 on a wearer. Alternatively, other fastening means, such as buttons, pins, snaps, adhesive tape fasteners, cohesives, mushroom-and-loop fasteners, a belt, and so forth, as well as combinations including at least one of the foregoing fasteners can be employed. Additionally, more than two fasteners can be provided, particularly if the article 10 is to be provided in a prefastened configuration.

The article 10 can further include other layers between the absorbent core 24 and the bodyside liner 22 or outer cover 20. For example, he article 10 can also include a surge management layer 34 located between the bodyside liner 22 and the absorbent core 24 to prevent pooling of the fluid exudates and further improve air exchange and distribution of the fluid exudates within the article 10.

The article 10 can be of various suitable shapes. For example, the article 10 can have an overall rectangular shape, T-shape or an approximately hourglass shape. In the shown aspect, the article 10 has a generally I-shape. The article 10 further defines a longitudinal direction 36 and a transverse direction 38. Other suitable article components that can be incorporated on absorbent articles include containment flaps, waist flaps, elastomeric side panels, and the like.

The various components of the article 10 are integrally assembled employing various types of attachment mechanisms such as adhesive, sonic bonds, thermal bonds, and so forth, as well as combinations including at least one of foregoing mechanisms. In the shown aspect, for example, the bodyside liner 22 and outer cover 20 are assembled to the absorbent core 24 with lines of adhesive, such as a hot melt, pressure-sensitive adhesive. Similarly, other article components, such as the elastic members 26 and 28, fastening members 30, and surge layers 34 can be assembled into the article 10 by employing the above-identified attachment mechanisms.

The outer cover 20 of the article 10 can include any material used for such applications, such as a substantially vapor-permeable material. The permeability of the outer cover 20 can be configured to enhance the breathability of the article 10 and to reduce the hydration of the wearer's skin during use without allowing excessive condensation of vapor, such as urine, on the garment facing surface of the outer cover 20 that can undesirably dampen the wearer's clothes. The outer cover 20 can be constructed to be permeable to at least water vapor and can have a water vapor transmission rate of greater than or equal to about 1,000 grams per square meter per 24 hours ($g/m^2/24$ hr). For example, the outer cover 20 can define a water vapor transmission rate of about 1,000 to about 6,000 $g/m^2/24$ hr.

The outer cover 20 is also desirably substantially liquid impermeable. For example, the outer cover 20 can be constructed to provide a hydrohead value of greater than or equal to about 60 centimeters (cm), or, more specifically, greater than or equal to about 80 cm, and even more specifically, greater than or equal to about 100 cm. A suitable technique for determining the resistance of a material to liquid penetration is Federal Test Method Standard (FTMS) 191 Method 5514, dated December 31, 1968.

As stated above, the outer cover 20 can include any material used for such applications, and desirably includes materials that either directly provide the above desired levels of liquid impermeability and air permeability and/or materials that can be modified or treated in some manner to provide such levels. The outer cover 20 can be a nonwoven fibrous web constructed to provide the required level of liquid impermeability. For example, a nonwoven web including spunbond and/or meltblown polymer fibers can be selectively treated with a water repellent coating and/or laminated with a liquid impermeable, vapor permeable polymer film to provide the outer cover 20. In another aspect, the outer cover 20 can include a nonwoven web including a plurality of randomly deposited hydrophobic thermoplastic meltblown fibers that are sufficiently bonded or otherwise connected to one another to provide a substantially vapor permeable and substantially liquid impermeable web. The outer cover 20 can also include a vapor permeable nonwoven layer that has been partially coated or otherwise configured to provide liquid impermeability in selected areas. In yet another example, the outer cover 20 is provided by an extensible material. Further, the outer cover 20 material can have stretch in the longitudinal 36 and/or transverse 38 directions. When the outer cover 20 is made from extensible or stretchable materials, the article 10 provides additional benefits to the wearer including improved fit.

The bodyside liner 22, employed to help isolate the wearer's skin from liquids held in the absorbent core 24, can define a compliant, soft, non-irritating feel to the wearer's skin. Further, the bodyside liner 22 can be less hydrophilic than the absorbent core 24, to present a relatively dry surface to the wearer, and can be sufficiently porous to be liquid permeable, permitting liquid to readily penetrate through its thickness. A suitable bodyside liner 22 can be manufactured from a wide selection of web materials, such as porous foams, reticulated foams, apertured plastic films, natural fibers (for example, wood or cotton fibers), synthetic fibers (for example, polyester or polypropylene fibers), and the like, as well as a combination of materials including at least one of the foregoing materials.

Various woven and nonwoven fabrics can be used for the bodyside liner 22. For example, the bodyside liner 22 can include a meltblown or spunbond web (e.g., of polyolefin fibers), a bonded-carded web (e.g., of natural and/or synthetic fibers), a substantially hydrophobic material (e.g., treated with a surfactant or otherwise processed to impart a desired level of wettability and hydrophilicity), and the like, as well as combinations including at least one of the foregoing. For example, the bodyside liner 22 can include a nonwoven, spunbond, polypropylene fabric, optionally including about 2.8 to about 3.2 denier fibers formed into a web having a basis weight of about 22 grams per square meter (g/m$^2$) and a density of about 0.06 gram per cubic centimeter (g/cc).

The absorbent core 24 of the article 10 can include a matrix of hydrophilic fibers, such as a fibrous web of cellulosic fibers, mixed with particles of the particulate superabsorbent polymer composition. The wood pulp fluff can be exchanged with synthetic, polymeric, meltblown fibers, and the like, as well as a combination including at least one of the foregoing. The particulate superabsorbent polymer composition can be substantially homogeneously mixed with the hydrophilic fibers or can be nonuniformly mixed. Alternatively, the absorbent core 24 can include a laminate of fibrous webs and particulate superabsorbent polymer composition and/or a suitable matrix for maintaining the particulate superabsorbent polymer composition in a localized area. When the absorbent core 24 includes a combination of hydrophilic fibers and the particulate superabsorbent polymer, the hydrophilic fibers and particulate superabsorbent polymer composition can form an average basis weight for the absorbent core 24 that can be about 400 grams per square meter (g/m$^2$) to about 900 g/m$^2$, or, more specifically, about 500 g/m$^2$ to about 800 g/m$^2$, and even more specifically, about 550 g/m$^2$ to about 750 g/m$^2$.

In general, the particulate superabsorbent polymer composition is present in the absorbent core 24 in an amount of greater than or equal to about 50 weight percent (wt%), or, more desirably greater than or equal to about 70wt%, based on a total weight of the absorbent core 24. For example, in a particular aspect, the absorbent core 24 can include a laminate that includes greater than or equal to about 50wt% , or, more desirably, greater than or equal to about 70wt% of particulate superabsorbent polymer overwrapped by a fibrous web or other suitable material for maintaining the high-absorbency material in a localized area.

Optionally, the absorbent core 24 can further include a support (e.g., a substantially hydrophilic tissue or nonwoven wrapsheet (not illustrated)) to help maintain the integrity of the structure of the absorbent core 24. The tissue wrapsheet can be placed about the web/sheet of high-absorbency material and/or fibers, optionally over at least one or both major facing surfaces thereof. The tissue wrapsheet can include an absorbent cellulosic material, such as creped wadding or a high wet-strength tissue. The tissue wrapsheet can optionally be configured to provide a wicking layer that helps to rapidly distribute liquid over the mass of absorbent fibers constituting the absorbent core 24. If this support is employed, the colorant 40 can optionally be disposed in the support, on the side of the absorbent core 24 opposite the outer cover 20.

Due to the thinness of absorbent core 24 and the high absorbency material within the absorbent core 24, the liquid uptake rates of the absorbent core 24, by itself, can be too low, or cannot be adequately sustained over multiple insults of liquid into the absorbent core 24. To improve the overall liquid uptake and air exchange, the article 10 can further include a porous, liquid-permeable layer of surge management layer 34, as representatively illustrated in FIG 5. The surge management layer 34 is typically less hydrophilic than the absorbent core 24, and can have an operable level of density and basis weight to quickly collect and temporarily hold liquid surges, to transport the liquid from its initial entrance point and to substantially completely release the liquid to other parts of the absorbent core 24. This configuration can help prevent the liquid from pooling and collecting on the portion of the article 10 positioned against the wearer's skin, thereby reducing the feeling of wetness by the wearer. The structure of the surge management layer 34 can also enhance the air exchange within the article 10.

Various woven and nonwoven fabrics can be used to construct the surge management layer 34. For example, the surge management layer 34 can be a layer including a meltblown or spunbond web of synthetic fibers (such as polyolefin fibers); a bonded-carded-web or an airlaid web including, for example, natural and/or synthetic fibers; hydrophobic material that is optionally treated with a surfactant or otherwise processed to impart a desired level of wettability and hydrophilicity; and the like, as well as combinations including at least one of the foregoing. The bonded carded-web can, for example, be a thermally bonded web that is bonded using low melt binder fibers, powder, and/or adhesive. The layer can optionally include a mixture of different fibers. For example, the surge management layer 34 can include a hydrophobic, nonwoven material having a basis weight of about 30 to about 120 g/m$^2$.

**TEST PROCEDURES**

**The Vortex Test**

The Vortex Test measures the amount of time in seconds required for 2 grams of a SAP to close a vortex created by stirring 50 milliliters of saline solution at 600 revolutions per minute on a magnetic stir plate. The time it takes for the vortex to close is an indication of the free swell absorbing rate of the SAP.

Equipment and materials

1. Schott Duran 100 ml Beaker and 50ml graduated cylinder.
2. Programmable magnetic stir plate, capable of providing 600 revolutions per minute (such as that commercially available from PMC Industries, under the trade designation Dataplate® Model #721).
3. Magnetic stir bar without rings, 7.9 millimeters.times.32 millimeters, Teflon® covered (such as that commercially available from Baxter Diagnostics, under the trade designation S/PRIM. brand single pack round stirring bars with removable pivot ring).
4. Stopwatch
5. Balance, accurate to +/- 0.01g
6. Saline solution, 0.87 w/w % Blood Bank Saline available from Baxter Diagnostics (considered, for the purposes of this application to be the equivalent of 0.9 wt. % saline
7. Weighing paper
8. Room with standard condition atmosphere: Temp = 23°C +/- 1°C. and Relative Humidity = 50% +/- 2%.

Test Procedure

1. Measure 50ml +/- 0.01ml of saline solution into the 100 ml beaker.
2. Place the magnetic stir bar into the beaker.
3. Program the magnetic stir plate to 600 revolutions per minute.
4. Place the beaker on the center of the magnetic stir plate such that the magnetic stir bar is activated. The bottom of the vortex should be near the top of the stir bar.
5. Weigh out 2g +/- 0.01g of the SAP to be tested on weighing paper.
NOTE: The SAP is tested as received (i.e. as it would go into an absorbent composite such as those described herein). No screening to a specific particle size is done, though the particle size is known to have an effect on this test.
6. While the saline solution is being stirred, quickly pour the SAP to be tested into the saline solution and start the stopwatch. The SAP to be tested should be added to the saline solution between the center of the vortex and the side of the beaker.
7. Stop the stopwatch when the surface of the saline solution becomes flat and record the time.
8. The time, recorded in seconds, is reported as the Vortex Time.

**Centrifuge Retention Capacity Test (CRC)**

The CRC Test measures the ability of the particulate SAP composition to retain liquid therein after being saturated and subjected to centrifugation under controlled conditions. The resultant retention capacity is stated as grams of liquid retained per gram weight of the sample, (g/g). The SAP sample to be tested is prepared from particles that are pre-screened through a U.S. standard 30-mesh screen and retained on a U.S. standard 50-mesh screen. As a result, the particulate SAP sample comprises particles sized in the range of about 300 to about 600 microns. The particles can be pre-screened by hand or automatically.

The retention capacity is measured by placing about 0.20 grams of the pre-screened particulate SAP sample into a water-permeable bag that will contain the sample while allowing a test solution (0.9 weight percent sodium chloride in distilled water) to be freely absorbed by the sample. A heat-sealable tea bag material, such as that available from Dexter Corporation (having a place of business in Windsor Locks, Connecticut, U.S.A.) as model designation 1234T heat sealable filter paper works well for most applications. The bag is formed by folding a 5-inch by 3-inch sample of the bag material in half and heat-sealing two of the open edges to form a 2.5-inch by 3-inch rectangular pouch. The heat seals are about 0.25 inches inside the edge of the material. After the sample is placed in the pouch, the remaining open edge of the pouch is also heat-sealed. Empty bags are also made to serve as controls. Three samples are prepared for each particulate SAP to be tested.

The sealed bags are submerged in a pan containing the test solution at about 23°C, making sure that the bags are held down until they are completely wetted. After wetting, the particulate SAP samples remain in the solution for about 30 minutes, at which time they are removed from the solution and temporarily laid on a non-absorbent flat surface.

The wet bags are then placed into the basket wherein the wet bags are separated from each other and are placed at the outer circumferential edge of the basket, wherein the basket is of a suitable centrifuge capable of subjecting the samples to a g-force of about 350. One suitable centrifuge is a CLAY ADAMS DYNAC II, model #0103, having a water collection basket, a digital rpm gauge, and a machined drainage basket adapted to hold and drain the flat bag samples. Where multiple samples are centrifuged, the samples are placed in opposing positions within the centrifuge to balance the basket when spinning. The bags (including the wet, empty bags) are centrifuged at about 1,600rpm (e.g., to achieve a target g-force of about 350g force with a variance from about 240 to about 360g force), for 3 minutes. G force is defined as an unit of inertial force on a body that is subjected to rapid acceleration or gravity, equal to 32 ft/sec$^2$ at sea level. The bags are removed and weighed, with the empty bags (controls) being weighed first, followed by the bags containing the particulate SAP samples. The amount of solution retained by the particulate SAP sample, taking into account the solution retained by the bag itself, is the centrifuge retention capacity (CRC) of the SAP, expressed as grams of fluid per gram of SAP. More particularly, the retention capacity is determined by the following equation:

$$\underline{CRC = [\text{sample bag after centrifuge} - \text{empty bag after centrifuge} - \text{dry sample}}$$

$$\underline{\text{weight}]}/\text{dry sample weight}$$

The three samples are tested, and the results are averaged to determine the CRC of the particulate SAP.

**Free-Swell Gel Bed Permeability Test (FSGBP)**

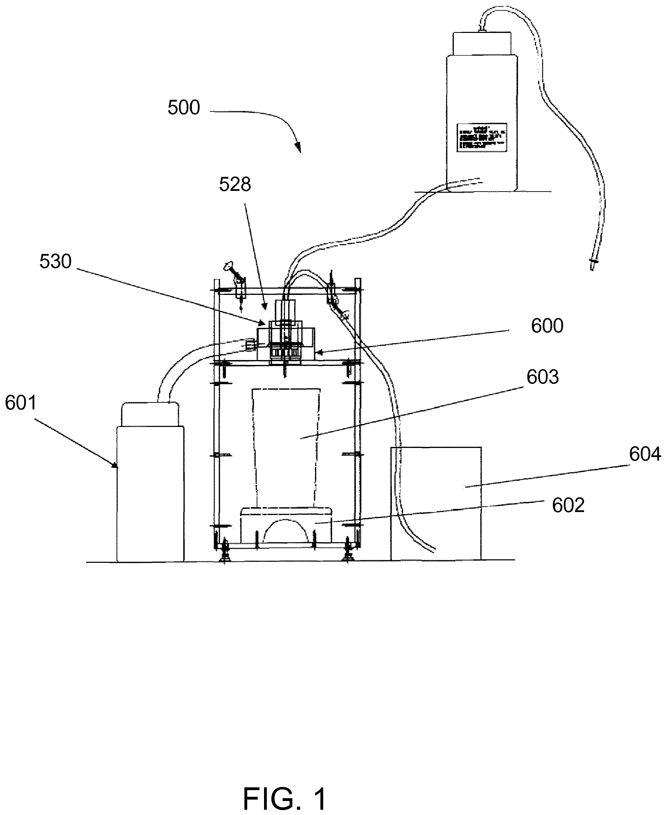

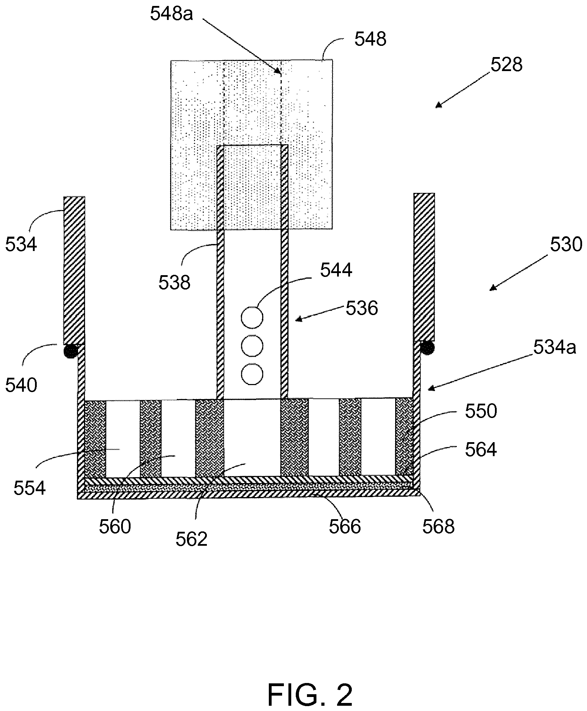

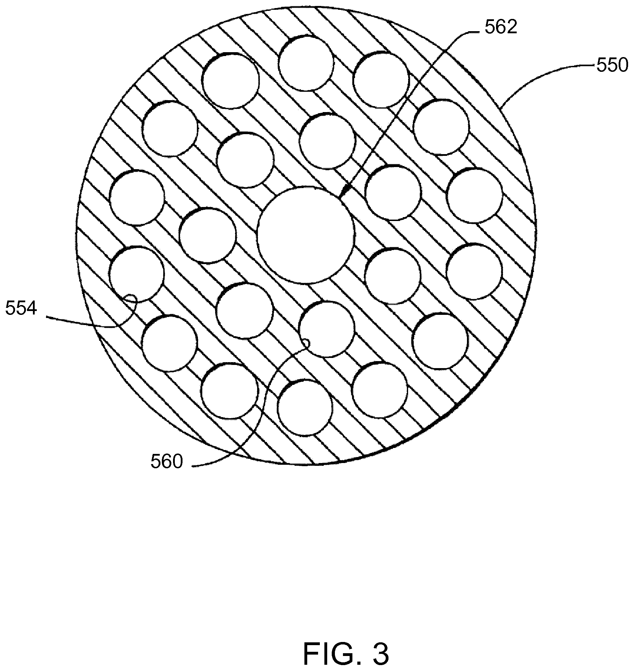

As used herein, the Free-Swell Gel Bed Permeability Test, also referred to as the Gel Bed Permeability Under 0 psi Swell Pressure Test (FSGBP), determines the permeability of a swollen bed of gel particles (e.g., such as the particulate SAP, or the particulate SAP prior to being surface treated), under what is commonly referred to as "free swell" conditions. The term "free swell" means that the gel particles are allowed to swell without a restraining load upon absorbing test solution as will be described. A suitable apparatus for conducting the Gel Bed Permeability Test is shown in FIGs 1, 2, and 3 and indicated generally as 500. The test apparatus assembly 528 comprises a sample container, generally indicated at 530, and a plunger, generally indicated at 536. The plunger comprises a shaft 538 having a cylinder hole bored down the longitudinal axis and a head 550 positioned at the bottom of the shaft. The shaft hole 562 has a diameter of about 16 mm. The plunger head is attached to the shaft, such as by adhesion. Twelve holes 544 are bored into the radial axis of the shaft, three positioned at every 90 degrees having diameters of about 6.4 mm. The shaft 538 is machined from a LEXAN rod or equivalent material and has an outer diameter of about 2.2 cm and an inner diameter of about 16 mm.
The plunger head 550 has a concentric inner ring of seven holes 560 and an outer ring of 14 holes 554, all holes having a diameter of about 8.8 millimeters as well as a hole of about 16 mm aligned with the shaft. The plunger head 550 is machined from a LEXAN rod or equivalent material and has a height of approximately 16 mm and a diameter sized such that it fits within the cylinder 534 with minimum wall clearance but still slides freely. The total length of the plunger head 550 and shaft 538 is about 8.25 cm, but can be machined at the top of the shaft to obtain the desired mass of the plunger 536. The plunger 536 comprises a 100 mesh stainless steel cloth screen 564 that is biaxially stretched to tautness and attached to the lower end of the plunger 536. The screen is attached to the plunger head 550 using an appropriate solvent that causes the screen to be securely adhered to the plunger head 550. Care must be taken to avoid excess solvent migrating into the open portions of the screen and reducing the open area for liquid flow. Acrylic adhesive, Weld-On #4, from IPS Corporation (having a place of business in Gardena, California, USA) is a suitable adhesive.
The sample container 530 comprises a cylinder 534 and a 400 mesh stainless steel cloth screen 566 that is biaxially stretched to tautness and attached to the lower end of the cylinder 534. The screen is attached to the cylinder using an appropriate solvent that causes the screen to be securely adhered to the cylinder. Care must be taken to avoid excess solvent migrating into the open portions of the screen and reducing the open area for liquid flow. Acrylic adhesive, Weld-On #4, from IPS Corporation is a suitable adhesive. A gel particle sample, indicated as 568 in FIG 2, is supported on the screen 566 within the cylinder 534 during testing.
The cylinder 534 may be bored from a transparent LEXAN rod or equivalent material, or it may be cut from a LEXAN tubing or equivalent material, and has an inner diameter of about 6 cm (e.g., a cross-sectional area of about 28.27 cm$^2$), a wall thickness of about 0.5 cm and a height of approximately 7.95 cm. A step is machined into the outer diameter of the cylinder 534 such that a region 534a with an outer diameter of 66 mm exists for the bottom 31 mm of the cylinder 534. An o-ring 540 which fits the diameter of region 534a may be placed at the top of the step.
The annular weight 548 has a counter-bored hole about 2.2 cm in diameter and 1.3 cm deep so that it slips freely onto the shaft 538. The annular weight also has a thrubore 548a of about 16 mm. The annular weight 548 can be made from stainless steel or from other suitable materials resistant to corrosion in the presence of the test solution, which is 0.9 weight percent sodium chloride solution in distilled water. The combined weight of the plunger 536 and annular weight 548 equals approximately 596 grams (g), which corresponds to a pressure applied to the sample 568 of about 0.3 pounds per square inch (psi), or about 20.7 dynes/cm$^2$ (2.07 kPa), over a sample area of about 28.27 cm$^2$.
When the test solution flows through the test apparatus during testing as described below, the sample container 530 generally rests on a weir 600. The purpose of the weir is to divert liquid that overflows the top of the sample container 530 and diverts the overflow liquid to a separate collection device 601. The weir can be positioned above a scale 602 with a beaker 603 resting on it to collect saline solution passing through the swollen sample 568.

To conduct the Gel Bed Permeability Test under "free swell" conditions, the plunger 536, with the weight 548 seated thereon, is placed in an empty sample container 530 and the height from the top of the weight 548 to the bottom of the sample container 530 is measured using a suitable gauge accurate to 0.01 mm. The force the thickness gauge applies during measurement should be as low as possible, preferably less than about 0.74 Newtons. It is important to measure the height of each empty sample container 530, plunger 536, and weight 548 combination and to keep track of which plunger 536 and weight 548 is used when using multiple test apparatus. The same plunger 536 and weight 548 should be used for measurement when the sample 568 is later swollen following saturation. It is also desirable that the base that the sample cup 530 is resting on is level, and the top surface of the weight 548 is parallel to the bottom surface of the sample cup 530.

The sample to be tested is prepared from the particulate SAP, which is prescreened through a U.S. standard 30 mesh screen and retained on a U.S. standard 50 mesh screen. As a result, the test sample comprises particles sized in the range of about 300 to about 600 microns. The SAP particles can be pre-screened with, for example, a RO-TAP Mechanical Sieve Shaker Model B available from W. S. Tyler, Inc., Mentor Ohio. Sieving is conducted for 10 minutes. Approximately 2.0 grams of the sample is placed in the sample container 530 and spread out evenly on the bottom of the sample container. The container, with 2.0 grams of sample in it, without the plunger 536 and weight 548 therein, is then submerged in the 0.9% saline solution for a time period of about 60 minutes to saturate the sample and allow the sample to swell free of any restraining load. During saturation, the sample cup 530 is set on a mesh located in the liquid reservoir so that the sample cup 530 is raised slightly above the bottom of the liquid reservoir. The mesh does not inhibit the flow of saline solution into the sample cup 530. A suitable mesh can be obtained as part number 7308 from Eagle Supply and Plastic, having a place of business in Appleton, Wisconsin, U.S.A. Saline does not fully cover the SAP particles, as would be evidenced by a perfectly flat saline surface in the test cell. Also, saline depth is not allowed to fall so low that the surface within the cell is defined solely by swollen SAP, rather than saline.

At the end of this period, the plunger 536 and weight 548 assembly is placed on the saturated sample 568 in the sample container 530 and then the sample container 530, plunger 536, weight 548, and sample 568 are removed from the solution. After removal and before being measured, the sample container 530, plunger 536, weight 548, and sample 568 are to remain at rest for about 30 seconds on a suitable flat, large grid non-deformable plate of uniform thickness. The thickness of the saturated sample 568 is determined by again measuring the height from the top of the weight 548 to the bottom of the sample container 530, using the same thickness gauge used previously provided that the zero point is unchanged from the initial height measurement. The sample container 530, plunger 536, weight 548, and sample 568 may be placed on a flat, large grid non-deformable plate of uniform thickness that will provide for drainage. The plate has an overall dimension of 7.6cm by 7.6cm, and each grid has a cell size dimension of 1.59cm long by 1.59cm wide by 1.12cm deep. A suitable flat, large grid non-deformable plate material is a parabolic diffuser panel, catalogue number 1624K27, available from McMaster Carr Supply Company, having a place of business in Chicago, Illinois, U.S.A., which can then be cut to the proper dimensions. This flat, large mesh non-deformable plate must also be present when measuring the height of the initial empty assembly. The height measurement should be made as soon as practicable after the thickness gauge is engaged. The height measurement obtained from measuring the empty sample container 530, plunger 536, and weight 548 is subtracted from the height measurement obtained after saturating the sample 568. The resulting value is the thickness, or height "H" of the swollen sample.

The permeability measurement is initiated by delivering a flow of the 0.9% saline solution into the sample container 530 with the saturated sample 568, plunger 536, and weight 548 inside. The flow rate of test solution into the container is adjusted to cause saline solution to overflow the top of the cylinder 534 thereby resulting in a consistent head pressure equal to the height of the sample container 530. The test solution may be added by any suitable means that is sufficient to ensure a small, but consistent amount of overflow from the top of the cylinder, such as with a metering pump 604. The overflow liquid is diverted into a separate collection device 601. The quantity of solution passing through the sample 568 versus time is measured gravimetrically using the scale 602 and beaker 603. Data points from the scale 602 are collected every second for at least sixty seconds once the overflow has begun. Data collection may be taken manually or with data collection software. The flow rate, Q, through the swollen sample 568 is determined in units of grams/second (g/s) by a linear least-square fit of fluid passing through the sample 568 (in grams) versus time (in seconds).

Permeability in $cm^2$ is obtained by the following equation:

$$K=[Q*H*\mu]/[A*\rho*P]$$

where K=Permeability ($cm^2$), Q=flow rate (g/sec), H=height of swollen sample (cm), $\mu$=liquid viscosity (poise) (approximately one centipoise for the test solution used with this Test), A=cross-sectional area for liquid flow (28.27 $cm^2$ for the

sample container used with this Test), $\rho$=liquid density (g/cm$^3$) (approximately one g/cm$^3$, for the test solution used with this Test) and P=hydrostatic pressure (dynes/cm$^2$) (normally approximately 7,797 dynes/cm$^2$). The hydrostatic pressure is calculated from $P=\rho *g*h,$ where $\rho$=liquid density (g/cm$^3$), g=gravitational acceleration, nominally 981 cm/sec$^2$, and h=fluid height, e.g., 7.95 cm for the Gel Bed Permeability Test described herein.

A minimum of two samples is tested and the results are averaged to determine the gel bed permeability of the sample of particulate SAP.

The FSGBP can be measured as described herein prior to subjecting a particulate SAP to a Processing Test as described herein. Such a FSGBP value can be referred to as the "original" FSGBP of the particulate SAP. The FSGBP may also be measured subsequent to subjecting the particulate SAP to the Processing Test. Such a FSGBP value can be referred to as the "post processing" FSGBP. Comparing the original FSGBP of a particulate SAP with the post processing FSGBP of the particulate SAP can be used as a measure of the stability of the composition. It should be noted that all "original" and "post processing" FSGBP values reported herein were measured using a sample of pre-screened 300 to 600 $\mu$m particles.

**Absorbency Under Load Test (AUL(0.9psi))**

The Absorbency Under Load (AUL) Test measures the ability of the particulate SAP to absorb a 0.9 weight percent solution of sodium chloride in distilled water at room temperature (test solution) while the material is under a 0.9 psi load. The apparatus for testing AUL consists of:

- An AUL assembly including a cylinder, a 4.4g piston, and a standard 317gm weight. The components of this assembly are described in additional detail below.
- A flat-bottomed square plastic tray that is sufficiently broad to allow the glass frits to lay on the bottom without contact with the tray walls. A plastic tray that is 9" by 9"(22.9cm x 22.9cm), with a depth of 0.5 to 1"(1.3cm to 2.5cm) is commonly used for this test method.
- A 9 cm diameter sintered glass frit with a 'C' porosity (25-50 microns). This frit is prepared in advance through equilibration in saline (0.9% sodium chloride in distilled water, by weight). In addition to being washed with at least two portions of fresh saline, the frit must be immersed in saline for at least 12 hours prior to AUL measurements.
- Whatman Grade 1, 9 cm diameter filter paper circles.
- A supply of saline (0.9% sodium chloride in distilled water, by weight).

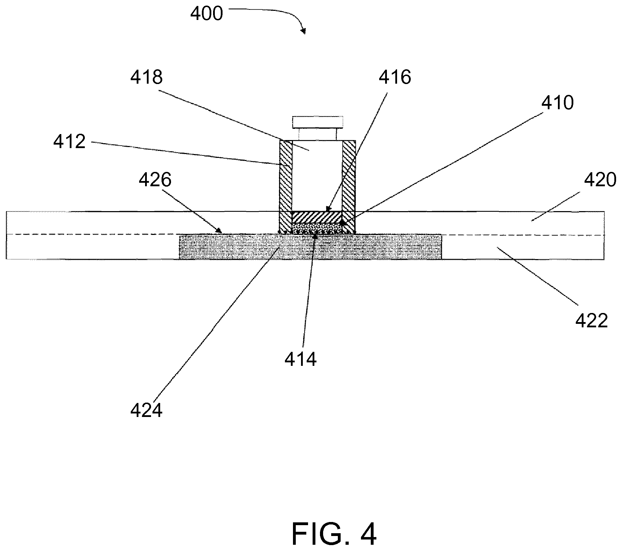

Referring to FIG 4, the cylinder 412 of the AUL assembly 400 used to contain the particulate superabsorbent polymer composition 410 is made from one-inch (2.54cm) inside diameter thermoplastic tubing machined-out slightly to be sure of concentricity. After machining, a 400 mesh stainless steel wire cloth 414 is attached to the bottom of the cylinder 412 by heating the steel wire cloth 414 in a flame until red hot, after which the cylinder 412 is held onto the steel wire cloth until cooled. A soldering iron can be utilized to touch up the seal if unsuccessful or if it breaks. Care must be taken to maintain a flat smooth bottom and not distort the inside of the cylinder 412.

The 4.4g piston (416) is made from one-inch diameter solid material (e.g., PLEXIGLAS®) and is machined to closely fit without binding in the cylinder 412.

A standard 317gm weight 418 is used to provide a 62,053dyne/cm$^2$ (about 0.9psi) restraining load. The weight is a cylindrical, 1 inch (2.5cm) diameter, stainless steel weight that is machined to closely fit without binding in the cylinder.

Unless specified otherwise, a sample 410 corresponding to a layer of at least about 300 gsm. (0.16g) of SAP particles is utilized for testing the AUL. The sample 410 is taken from SAP particles that are pre-screened through U.S. standard #30 mesh and retained on U.S. std. #50 mesh. The SAP particles can be pre-screened with, for example, a RO-TAP® Mechanical Sieve Shaker Model B available from W. S. Tyler, Inc., Mentor Ohio. Sieving is conducted for about 10 minutes.

The inside of the cylinder 412 is wiped with an antistatic cloth prior to placing the SAP particles 410 into the cylinder 412.

The desired amount of the sample of sieved particulate SAP 410 (about 0.16g) is weighed out on a weigh paper and evenly distributed on the wire cloth 414 at the bottom of the cylinder 412. The weight of the particulate SAP in the bottom of the cylinder is recorded as 'SA,' for use in the AUL calculation described below. Care is taken to be sure no particulate SAP cling to the wall of the cylinder. After carefully placing the 4.4g piston 412 and 317g weight 418 on the SAP particles 410 in the cylinder 412, the AUL assembly 400 including the cylinder, piston, weight, and SAP particles is weighed, and the weight is recorded as weight 'A'.

A sintered glass frit 424 (described above) is placed in the plastic tray 420, with saline 422 added to a level equal to that of the upper surface of the glass frit 424. A single circle of filter paper 426 is placed gently on the glass frit 424, and the AUL assembly 400 with the particulate SAP 410 is then placed on top of the filter paper 426. The AUL assembly 400 is then allowed to remain on top of the filter paper 426 for a test period of one hour, with attention paid to keeping the saline level in the tray constant. At the end of the one hour test period, the AUL apparatus is then weighed, with this value recorded as weight 'B.'

The AUL(0.9psi) is calculated as follows:

$$AUL(0.9psi) = (B-A)/SA$$

wherein

A= Weight of AUL Unit with dry SAP
B= Weight of AUL Unit with SAP after 60 minutes absorption
SA = Actual SAP weight

A minimum of two tests is performed and the results are averaged to determine the AUL value under 0.9psi load. The particulate SAP samples are tested at about 23°C and about 50% relative humidity.

### PAI Test

The Pressure Absorbency Index is the sum of the Absorbency Under Load values (described herein below) for a SAP determined under the following loads: 0.01 pound per square inch (690 dynes per square centimeter); 0.29 pound per square inch (19995 dynes per square centimeter); 0.57 pound per square inch (39300 dynes per square centimeter); and 0.90 pound per square inch (62053 dynes per square centimeter). That is, the Absorbency Under Load values for a given SAP are determined under the restraining forces set forth above according to the method set forth below in connection with the examples. The Absorbency Under Load values determined under the restraining loads set forth above are then totaled to produce the Pressure Absorbency Index.

### Moisture Content Test

The amount of water content, measured as "% moisture," can be measured as follows: 1) Weigh 5.0 grams of superabsorbent polymer composition (SAP) accurately in a pre-weighed aluminum weighing pan; 2) place the SAP and pan into a standard lab oven preheated to 105°C for 3 hours; 3) remove and re-weigh the pan and contents; and 4) calculate the percent moisture using the following formula:

$$\% \text{ Moisture} = \{((\text{pan wt} + \text{initial SAP wt}) - (\text{dried SAP \& pan wt})) * 100\} / \text{initial SAP wt}$$

EXAMPLES

The following Comparative Examples 1-4, and Examples 1-12 and Tables 1 and 2 are provided to illustrate the inventions of products including particulate superabsorbent polymer and processes to make particulate superabsorbent polymer as set forth in the claims, and do not limit the scope of the claims. Unless otherwise stated all parts, and percentages are based on the dry particulate superabsorbent polymer.

**Example 1** Into a polyethylene vessel equipped with an agitator and cooling coils was added, 2.0kg of 50% NaOH and 3.32kg of deionized water and cooled to 20 °C. 0.8kg of glacial acrylic acid was then added to the caustic solution and the solution again cooled to 20°C. 0.6g of polyethylene glycol monoallylether acrylate, 1.2g of ethoxylated trimethylol propane triacrylate SARTOMER® 9035 product, and 1.6kg of glacial acrylic acid were added to the first solution, followed by cooling to 4-6°C. Nitrogen was bubbled through the monomer solution for about 5 minutes. Dissolve 4.38g sodium bicarbonate, 0.0364g Tween 80 and 0.0364g Span20 in 95.55g of water. Add the mixture to the monomer solution and mix it with Silverson High Shear Mixer at 6500 RPM for 30 seconds. The monomer solution was then discharged into a rectangular tray. 80g of 1% by weight of $H_2O_2$ aqueous solution, 120g of 2wt% aqueous sodium persulfate solution, and 72g of 0.5wt% aqueous sodium erythorbate solution was added into the monomer solution to initiate polymerization reaction. The agitator was stopped and the initiated monomer was allowed to polymerize for 20 minutes.

The resulting hydrogel was chopped and extruded with a Hobart 4M6 commercial extruder, followed by drying in a Procter & Schwartz Model 062 forced air oven at 175°C for 12 minutes with up flow and 6 minutes with down flow air on a 20inch × 40inch perforated metal tray to a final product moisture level of less than 5 wt%. The dried material was coarse-ground in a Prodeva Model 315-S crusher, milled in an MPI 666-F three-stage roller mill and sieved with a Minox MTS 600DS3V to remove particles greater than 850$\mu$m and smaller than 150$\mu$m.

8 g of ethylene carbonate solution (50% wt/wt in water) were applied on the surface of 400 g of the above particles. A using a finely atomized spray from a Paasche VL sprayer while the superabsorbent polymer particles were fluidized in air and continuously mixed. The coated material was then heated in a convection oven at 185°C for 55 minutes for surface crosslinking. The surface crosslinked particulate material was then sieved with 20/100 mesh US standard sieves to remove particles greater than $850\mu m$ and smaller than $150\mu m$. The surface crosslinked particulate material was cooled down to below 60 °C and coated with a solution containing 0.4 g of polyethylene glycol (molecular weight 8000) and 40 g of deionized water. The coated material was relaxed at room temperature for one day and then sieved with 20/100 mesh US standard sieves to remove particles greater than $850\mu m$ and smaller than $150\mu m$.

The following is a representative of the PSD and average particle diameter (D50) of example 1:

Table 1

| | |
|---|---|
| % on 20 mesh (>850 microns) | 0.1 |
| % on 30 mesh (600-850 microns) | 7.6 |
| % on 50 mesh (300-600 microns) | 64.1 |
| % on 100 mesh (150-300 microns) | 22.2 |
| % on 140 mesh 106-150 microns) | 3.5 |
| % on 325 mesh (45-90 microns) | 2.4 |
| % thru 325 mesh (<45 microns) | 0.1 |
| D50 (microns) | 397 |

Example 2 - Same as Example 1, except that 0.0364g Tween 80 and 0.0364g Span20 were replaced with 0.0364g Tween 80 and 0.0364g Span40.

Example 3 - Same as Example 1, except that 0.0364g Tween 80 and 0.0364g Span20 were replaced with 0.0364g Tween 80 and 0.0364g Span60.

Example 4 - Same as Example 1, except that 0.0364g Tween 80 and 0.0364g Span20 were replaced with 0.0364g Tween 20 and 0.0364g Span20.

Example 5 - Same as Example 1, except that 0.0364g Tween 80 and 0.0364g Span20 were replaced with 0.0364g Tween 40 and 0.0364g Span20.

Example 6 - Same as Example 1, except that 0.0364g Tween 80 and 0.0364g Span20 were replaced with 0.0364g Tween 60 and 0.0364g Span20

Comparative Example 1 Control - regular surface crosslinked superabsorbent polymer without surfactant or foaming agent.

Comparative Example 2 - Includes a surfactant mixture of 0.0364g Tween 80 and 0.0364g Span20 but no foaming agent.

Comparative Example 3 - Example 1 which includes only 0.025% Span 20 but no Tween 80.

Comparative Example 4 - Example 1 which includes only 0.025% Tween 80 but no Span 20.

**Neutralized Aluminum Salt A**

200 g of aluminum sulfate solution (20% aqueous solution) was stirred in a beaker with a magnetic stirring bar. To this solution was added sodium hydroxide solution (50% aqueous solution) until the pH of the mixture reached 7. Totally 130 g of sodium hydroxide solution was consumed. The white colloidal suspension was stirred for 15 minutes and further sheared with Turnax mixer for about 1 minute to break down clumps. The neutralized aluminum solution was used for superabsorbent polymer modification without further purification.

Example 7 - Example 1 was changed wherein the surface crosslinked particulate material was cooled down to below 60 °C and coated with a solution containing 16g of Neutralized Aluminum Salt A and 0.4 g of polyethylene glycol (molecular weight 8000) and 40 g of deionized water. The coated material was relaxed at room temperature for one day and then sieved with 20/100 mesh US standard sieves to remove particles greater than $850\mu m$ and smaller than $150\mu m$.

Example 8 - 16 g of Neutralized Aluminum Salt A and 8 g of ethylene carbonate solution (50% wt/wt in water) were applied on the surface of 400 g of SAP of Example 1 using a finely atomized spray from a Paasche VL sprayer while the SAP particles were fluidized in air and continuously mixed. The coated material was then heated in a convection oven at 185°C for 55 minutes for surface crosslinking. The surface crosslinked particulate material was then sieved with 20/100 mesh US standard sieves to remove particles greater than $850\mu m$ and smaller than $150\mu m$. The surface

crosslinked particulate material was cooled down to below 60 °C and coated with a solution containing 16 g of Neutralized Aluminum Salt A and 0.4 g of polyethylene glycol (molecular weight 8000) and 40 g of deionized water. The coated material was relaxed at room temperature for one day and then sieved with 20/100 mesh US standard sieves to remove particles greater than 850μm and smaller than 150μm.

Example 9 -0.02wt% of ethylene acrylic acid thermoplastic polymer and 8 g of ethylene carbonate solution (50% wt/wt in water) were applied on the surface of 400 g of SAP of Example 1 using a finely atomized spray from a Paasche VL sprayer while the SAP particles were fluidized in air and continuously mixed.

Example 10 - The surface crosslinked particulate material of Example 1 was cooled down to below 60 °C and coated with a solution containing 0.2wt% of (pentasodium salt of diethylenetriaminepentacetic acid, Na5DTPA) and 0.4 g of polyethylene glycol (molecular weight 8000) and 40 g of deionized water. The coated material was relaxed at room temperature for one day and then sieved with 20/100 mesh US standard sieves to remove particles greater than 850μm and smaller than 150μm.

Example 11 - Example 1 was changed to add 0.5wt% of Kaolin clay to the hydrogel of Example 1.

Example 12 - Same as Example 1, except the 1.2g of ethoxylated trimethylol propane triacrylate SARTOMER® 9035 internal crosslinker is replaced by 1.705 gr Dynasylan®6490 polysiloxane (0.275% boaa), 0.744 gr of polyethylene glycol 300 diacrylate (Peg300DA) (0.120% boaa), 0.12 gr of polyethylene glycol monoallylether acrylate (PEGMAE) (0.120% boaa). The product of Example 12 has a CRC Increase of 2.7g/g.

Table 2

| Examples | CRC | 0.01 AUL | 0.3 AUL | 0.6 AUL | 0.9 AUL | PAI | Vortex |
|---|---|---|---|---|---|---|---|
| Example 1 | 39.3 | 54.6 | 37.1 | 28.7 | 18.6 | 139 | 44.6 |
| Example 2 | 38.4 | 53.3 | 36.6 | 28.2 | 18.9 | 137 | 50.2 |
| Example 3 | 39.1 | 54.1 | 37.1 | 28.5 | 18.2 | 137.9 | 51.3 |
| Example 4 | 38.4 | 53.2 | 36.8 | 28.1 | 17.9 | 136 | 54.6 |
| Example 5 | 37.9 | 54.1 | 36.5 | 27.7 | 18.1 | 136.4 | 52.8 |
| Example 6 | 39.2 | 53.6 | 37.4 | 27.6 | 18.5 | 137.1 | 50.1 |
| Comparative Ex 1 (Control) | 37.9 | 48.2 | 28.9 | 23.1 | 17.2 | 117.4 | 95.6 |
| Comparative Ex 2 | 39.5 | 46.5 | 27.8 | 22.8 | 16.1 | 113.2 | 81.2 |
| Comparative Ex 3 | 38.9 | 50.4 | 34.6 | 27.8 | 17.5 | 130.3 | 60.1 |
| Comparative Ex 4 | 38.6 | 50.6 | 33.9 | 27.5 | 17.6 | 129.6 | 62.5 |
| Example 7 | 37.5 | 52.1 | 37.2 | 28.9 | 18.5 | 136.7 | 41.5 |
| Example 8 | 37.9 | 53.1 | 37.5 | 28.1 | 18.5 | 137.2 | 42.5 |
| Example 9 | 38.2 | 53.4 | 38.2 | 27.9 | 18.1 | 137.6 | 44.1 |
| Example 10 | 38.1 | 52.7 | 38.6 | 27.6 | 17.8 | 136.7 | 44.5 |
| Example 11 | 38.5 | 53.1 | 36.5 | 27.3 | 17.9 | 134.8 | 41.1 |
| Example 12 | 33.5 | 48.9 | 34.3 | 26.8 | 18.2 | 128.15 | 52.6 |

## Claims

1. A process for making a particulate superabsorbent polymer having fast water absorption comprising the steps of

   a) preparing an aqueous monomer solution of a mixture of a of polymerizable unsaturated acid group containing monomer and an internal crosslinking agent monomer wherein the aqueous monomer solution comprises dissolved oxygen;
   b) sparging the aqueous monomer solution of step a) including adding an inert gas to the aqueous monomer solution of step a) to replace the dissolved oxygen of the aqueous monomer solution;
   c) polymerizing the aqueous monomer solution of step b) including the steps of

      c1) adding to the aqueous monomer solution of step a): i) an aqueous solution comprising from 0.05 to 2.0 wt.% based on the total amount of the polymerizable unsaturated acid group containing monomer solution of a foaming agent; and ii) an aqueous solution comprising from 0.001 to 1.0 wt.% based on the total amount of the polymerizable unsaturated acid group containing monomer solution of a mixture of a lipophile surfactant and a polyethoxylated hydrophilic surfactant;

c2) treating the monomer solution of step c1) to high speed shear mixing to form a treated monomer solution, wherein the components i) an aqueous solution comprising from 0.1 to 1.0 wt.% of a foaming agent; and ii) an aqueous solution comprising from 0.001 to 1.0 wt.% of a mixture of a lipophile surfactant and a polyethoxylated hydrophilic surfactant are added to the aqueous monomer solution after step b) of sparging the aqueous monomer solution and before step c2) of high speed shear mixing of the aqueous monomer solution;

c3) forming a hydrogel by adding a polymerization initiator to the treated monomer solution of step c2) wherein the initiator is added to the treated monomer solution after the foaming agent and the mixture of surfactants, wherein the polymer is formed to include bubbles of the foaming agent into the polymer structure;

d) drying and grinding the hydrogel of step c) to form particulate superabsorbent polymer; and
e) surface crosslinking the particulate superabsorbent polymer of step d) with a surface crosslinking agent wherein the surface crosslinked superabsorbent polymer has a vortex of from 30 sec to 60 sec,
whereby the superabsorbent polymer includes from 0.001wt% to 5wt% by weight based on the total amount of the polymerizable unsaturated acid group containing monomer of at least one internal crosslinking agent,
wherein the lipophile surfactant is nonionic and has a HLB of from 4 to 9 and the polyethoxylated hydrophilic surfactant is nonionic and has a HLB of from 12 to 18.

2. The process for making the particulate superabsorbent polymer of claim 1 wherein the mixture of a lipophile surfactant and a polyethoxylated hydrophilic surfactant has a HLB of from 8 to 14.

3. The process for making the particulate superabsorbent polymer of claim 1 wherein the lipophile surfactant is a sorbitan ester and the polyethoxylated hydrophilic surfactant is a polyethoxylated sorbitan ester.

4. The process for making the particulate superabsorbent polymer of claim 1 wherein the foaming agent is selected from an alkali metal carbonate or alkali metal bicarbonate.

5. The process for making the particulate superabsorbent polymer of claim 1 wherein the superabsorbent polymer has a Pressure Absorbency Index of from 120 to 150.

6. The process for making the particulate superabsorbent polymer of claim 1 comprising from 0.05 to 1.0 wt.% based on the total amount of the polymerizable unsaturated acid group containing monomer solution of the polymerization initiator.

7. The process for making the particulate superabsorbent polymer of claim 1 wherein said particulate superabsorbent polymer composition has particles having a particle diameters of smaller than 600μm and larger than 150μm in an amount of not less than 85wt% of the particulate superabsorbent polymer composition and as specified by standard sieve classification and the particles having a weight average particle diameter (D50) specified by standard sieve classification of from 300 to 400μm.

8. The process for making the particulate superabsorbent polymer of claim 1 further comprising the step of
e) mixing the surface crosslinked superabsorbent polymer with a chelating agent, wherein an amount of the chelating agent is from 0.001 to 10 weight parts per 100 weight parts of the particulate superabsorbent polymer.

9. The process for making the particulate superabsorbent polymer of claim 8 wherein the chelating agent is selected from aminocarboxylic acids with at least three carboxyl groups and their salts.

10. The process for making the particulate superabsorbent polymer of claim 1 comprising the step of adding from 0.01 to 0.5 % weight of a thermoplastic polymer based on dry polymer powder weight is applied on the particle surface wherein the thermoplastic polymer is either added to the particulate superabsorbent polymer with the surface crosslinking agent or applied to the particulate superabsorbent polymer before the surface crosslinking agent is added to the particulate superabsorbent polymer, and heat treating the coated superabsorbent polymer particle at a temperature between 150°C and 250°C for from 0.5 to 60 minutes to effectuate the surface crosslinking of the superabsorbent polymer particle.

11. The process for making the particulate superabsorbent polymer of claim 10 wherein the thermoplastic polymer is selected from polyethylene, polyesters, polyurethanes, linear low density polyethylene (LLDPE), ethylene acrylic acid copolymer (EAA), styrene copolymers, ethylene alkyl methacrylate copolymer (EMA), polypropylene (PP), ethylene vinyl acetate copolymer (EVA) or blends thereof, or copolymers thereof.

12. The process for making the particulate superabsorbent polymer of claim 10 wherein the thermoplastic polymer is added to the particulate superabsorbent polymer with the surface crosslinking agent.

13. The process for making the particulate superabsorbent polymer of claim 10 wherein the thermoplastic polymer is added to the particulate superabsorbent polymer before the surface crosslinking agent c) is added to the particulate superabsorbent polymer.

14. A particulate superabsorbent polymer comprising an internal crosslinking structure, from 0.05 to 2 wt.% based on the total amount of the polymerizable unsaturated acid group containing monomer solution of a foaming agent, and from 0.001 to 1.0 wt.% based on the total amount of the polymerizable unsaturated acid group containing monomer solution of a mixture of a lipophile nonionic surfactant and a polyethoxylated hydrophilic nonionic surfactant in an inside of the particle, the particle having a surface which has been subjected to a cross-linking treatment for cross-linking the surface, the particulate superabsorbent polymer having a Vortex time of from 30 to 60 seconds, whereby the average pore diameter of the particulate superabsorbent polymer is in the range of 10-500$\mu$m, whereby the average pore diameter is found by subjecting the cross section of the particulate superabsorbent polymer in a dry state to image analysis with the aid of an electron microscope, whereby the average pore diameter is obtained by forming a histogram representing the distribution of pore diameters of the particulate superabsorbent polymer in consequence of the image analysis and calculating the number average of pore diameters based on the histogram, wherein the lipophile nonionic surfactant has a HLB of from 4 to 9 and the polyethoxylated hydrophilic nonionic surfactant has a HLB of from 12 to 18.

15. The particulate superabsorbent polymer of claim 14 wherein the mixture of a lipophile nonionic surfactant and a polyethoxylated hydrophilic nonionic surfactant has a HLB of from 8 to 14.

16. The particulate superabsorbent polymer of claim 15 wherein the lipophile nonionic surfactant is a sorbitan ester and the polyethoxylated hydrophilic nonionic surfactant is a polyethoxylated sorbitan ester.

17. The particulate superabsorbent polymer of claim 14 wherein said particulate superabsorbent polymer composition has particles having a particle diameters of smaller than 600$\mu$m and larger than 150$\mu$m in an amount of not less than 85wt% of the particulate superabsorbent polymer composition and as specified by standard sieve classification and the particles having a weight average particle diameter (D50) specified by standard sieve classification of from 300 to 400$\mu$m.

18. An absorbent article comprising:

   a topsheet;
   backsheet;
   an absorbent core disposed between the topsheet and backsheet, the absorbent core comprising a particulate superabsorbent polymer composition comprising an internal crosslinking structure, from 0.05 to 2.0 wt.% based on the total amount of the polymerizable unsaturated acid group containing monomer solution of a foaming agent, and from 0.001 to 1.0 wt.% based on the total amount of the polymerizable unsaturated acid group containing monomer solution of a mixture of a lipophile nonionic surfactant and a polyethoxylated hydrophilic nonionic surfactant in an inside of the particle, wherein the lipophile nonionic surfactant has a HLB of from 4 to 9 and the polyethoxylated hydrophilic nonionic surfactant has a HLB of from 12 to 18, the particle having a surface which has been subjected to a cross-linking treatment for cross-linking the surface, the particulate superabsorbent polymer having a Vortex time of from 30 to 60 seconds, whereby the average pore diameter of the particulate superabsorbent polymer is in the range of 10-500$\mu$m, whereby the average pore diameter is found by subjecting the cross section of the particulate superabsorbent polymer in a dry state to image analysis with the aid of an electron microscope, whereby the average pore diameter is obtained by forming a histogram representing the distribution of pore diameters of the particulate superabsorbent polymer in consequence of the image analysis and calculating the number average of pore diameters based on the histogram.

**Patentansprüche**

1. Verfahren zur Herstellung eines partikelförmigen superabsorbierenden Polymers mit schneller Wasserabsorption, umfassend die Schritte:

   a) Herstellen einer wässrigen Monomerlösung aus einem Gemisch eines polymerisierbaren ungesättigten Säuregruppe-enthaltenden Monomers und eines internes-Vernetzungsmittel-Monomers, wobei die wässrige Monomerlösung gelösten Sauerstoff umfasst;
   b) Durchblasen der wässrigen Monomerlösung von Schritt a), umfassend Zugeben eines Inertgases zu der wässrigen Monomerlösung von Schritt a), um den gelösten Sauerstoff der wässrigen Monomerlösung zu ersetzen;
   c) Polymerisieren der wässrigen Monomerlösung von Schritt b), umfassend die Schritte:

   c1) Zugeben zu der wässrigen Monomerlösung von Schritt a) : i) eine wässrige Lösung, die von 0,05 bis 2,0 Gew.-%, bezogen auf die Gesamtmenge der Lösung des polymerisierbaren ungesättigten Säuregruppe-enthaltenden Monomers, an einem Schaumbildner umfasst; und ii) eine wässrige Lösung, die von 0,001 bis 1,0 Gew.-%, bezogen auf die Gesamtmenge der Lösung des polymerisierbaren ungesättigten Säuregruppe-enthaltenden Monomers, an einem Gemisch eines lipophilen grenzflächenaktiven Mittels und eines polyethoxylierten hydrophilen grenzflächenaktiven Mittels umfasst;
   c2) Behandeln der Monomerlösung von Schritt c1) durch hochscherendes Mischen, um eine behandelte Monomerlösung zu bilden, wobei die Komponenten i) eine wässrige Lösung, die von 0,1 bis 1,0 Gew.-% an einem Schaumbildner umfasst; und ii) eine wässrige Lösung, die von 0,001 bis 1,0 Gew.-% an einem Gemisch eines lipophilen grenzflächenaktiven Mittels und eines polyethoxylierten hydrophilen grenzflächenaktiven Mittels umfasst, zu der wässrigen Monomerlösung nach Schritt b) des Durchblasens der wässrigen Monomerlösung und vor Schritt c2) des hochscherenden Mischens der wässrigen Monomerlösung zugegeben werden;
   c3) Bilden eines Hydrogels durch Zugeben eines Polymerisationsinitiators zu der behandelten Monomerlösung von Schritt c2), wobei der Initiator nach dem Schaumbildner und dem Gemisch von grenzflächenaktiven Mitteln zu der behandelten Monomerlösung zugegeben wird, wobei das Polymer so gebildet wird, dass es Blasen des Schaumbildners in die Polymerstruktur einschließt;

   d) Trocknen und Mahlen des Hydrogels von Schritt c), um ein partikelförmiges superabsorbierendes Polymer zu bilden; und
   e) Oberflächenvernetzen des partikelförmigen superabsorbierenden Polymers von Schritt d) mit einem Oberflächenvernetzungsmittel, wobei das oberflächenvernetzte superabsorbierende Polymer einen Vortex von 30 s bis 60 s aufweist,
   wobei das superabsorbierende Polymer von 0,001 Gew.-% bis 5 Gew.-%, bezogen auf die Gesamtmenge des polymerisierbaren ungesättigten Säuregruppe-enthaltenden Monomers, an wenigstens einem internen Vernetzungsmittel enthält,
   wobei das lipophile grenzflächenaktive Mittel nichtionisch ist und einen HLB-Wert von 4 bis 9 aufweist und das polyethoxylierte hydrophile grenzflächenaktive Mittel nichtionisch ist und einen HLB-Wert von 12 bis 18 aufweist.

2. Verfahren zur Herstellung des partikelförmigen superabsorbierenden Polymers gemäß Anspruch 1, wobei das Gemisch eines lipophilen grenzflächenaktiven Mittels und eines polyethoxylierten hydrophilen grenzflächenaktiven Mittels einen HLB-Wert von 8 bis 14 aufweist.

3. Verfahren zur Herstellung des partikelförmigen superabsorbierenden Polymers gemäß Anspruch 1, wobei das lipophile grenzflächenaktive Mittel ein Sorbitanester ist und das polyethoxylierte hydrophile grenzflächenaktive Mittel ein polyethoxylierter Sorbitanester ist.

4. Verfahren zur Herstellung des partikelförmigen superabsorbierenden Polymers gemäß Anspruch 1, wobei der Schaumbildner ausgewählt ist aus einem Alkalimetallcarbonat oder Alkalimetallbicarbonat.

5. Verfahren zur Herstellung des partikelförmigen superabsorbierenden Polymers gemäß Anspruch 1, wobei das superabsorbierende Polymer einen Druckabsorptionsindex von 120 bis 150 aufweist.

6. Verfahren zur Herstellung des partikelförmigen superabsorbierenden Polymers gemäß Anspruch 1, umfassend von 0,05 bis 1,0 Gew.-%, bezogen auf die Gesamtmenge der Lösung des polymerisierbaren ungesättigten Säuregruppe-enthaltenden Monomers, an dem Polymerisationsinitiator.

7. Verfahren zur Herstellung des partikelförmigen superabsorbierenden Polymers gemäß Anspruch 1, wobei die partikelförmige superabsorbierende Polymerzusammensetzung Partikel mit Partikeldurchmessern von kleiner als 600 $\mu$m und größer als 150 $\mu$m in einer Menge von nicht weniger als 85 Gew.-% der partikelförmigen superabsorbierenden Polymerzusammensetzung und wie spezifiziert durch eine Standardsieb-Klassierung aufweist und die Partikel einen gewichtsgemittelten Partikeldurchmesser (D50), spezifiziert durch eine Standardsieb-Klassierung, von 300 bis 400 $\mu$m aufweisen.

8. Verfahren zur Herstellung des partikelförmigen superabsorbierenden Polymers gemäß Anspruch 1, ferner umfassend den Schritt:
e) Mischen des oberflächenvernetzten superabsorbierenden Polymers mit einem Chelatbildner, wobei die Menge des Chelatbildners von 0,001 bis 10 Gewichtsteile pro 100 Gewichtsteile des partikelförmigen superabsorbierenden Polymers beträgt.

9. Verfahren zur Herstellung des partikelförmigen superabsorbierenden Polymers gemäß Anspruch 8, wobei der Chelatbildner ausgewählt ist aus Aminocarbonsäuren mit wenigstens drei Carboxygruppen und deren Salzen.

10. Verfahren zur Herstellung des partikelförmigen superabsorbierenden Polymers gemäß Anspruch 1, umfassend den Schritt des Zugebens von 0,01 bis 0,5 Gew.-% an einem thermoplastischen Polymer, bezogen auf das Gewicht an trockenem Polymerpulver, das auf die Partikeloberfläche aufgebracht wird, wobei das thermoplastische Polymer mit dem Oberflächenvernetzungsmittel zu dem partikelförmigen superabsorbierenden Polymer zugegeben wird oder auf das partikelförmige superabsorbierende Polymer aufgebracht wird, bevor das Oberflächenvernetzungsmittel zu dem partikelförmigen superabsorbierenden Polymer zugegeben wird, und Wärmebehandeln des beschichteten superabsorbierenden Polymerpartikels bei einer Temperatur von zwischen 150 °C und 250 °C für 0,5 bis 60 Minuten, um das Oberflächenvernetzen des superabsorbierenden Polymerpartikels zu bewirken.

11. Verfahren zur Herstellung des partikelförmigen superabsorbierenden Polymers gemäß Anspruch 10, wobei das thermoplastische Polymer ausgewählt ist aus Polyethylen, Polyestern, Polyurethanen, linearem Polyethylen mit niedriger Dichte (LLDPE), EthylenAcrylsäure-Copolymer (EAA), Styrol-Copolymeren, Ethylen-Alkylmethacrylat-Copolymer (EMA), Polypropylen (PP), Ethylen-Vinylacetat-Copolymer (EVA) und Gemischen davon und Copolymeren davon.

12. Verfahren zur Herstellung des partikelförmigen superabsorbierenden Polymers gemäß Anspruch 10, wobei das thermoplastische Polymer mit dem Oberflächenvernetzungsmittel zu dem partikelförmigen superabsorbierenden Polymer zugegeben wird.

13. Verfahren zur Herstellung des partikelförmigen superabsorbierenden Polymers gemäß Anspruch 10, wobei das thermoplastische Polymer zu dem partikelförmigen superabsorbierenden Polymer zugegeben wird, bevor das Oberflächenvernetzungsmittel c) zu dem partikelförmigen superabsorbierenden Polymer zugegeben wird.

14. Partikelförmiges superabsorbierendes Polymer, umfassend eine interne Vernetzungsstruktur, von 0,05 bis 2 Gew.-%, bezogen auf die Gesamtmenge der Lösung des polymerisierbaren ungesättigten Säuregruppe-enthaltenden Monomers, an einem Schaumbildner und von 0,001 bis 1,0 Gew.-%, bezogen auf die Gesamtmenge der Lösung des polymerisierbaren ungesättigten Säuregruppe-enthaltenden Monomers, an einem Gemisch eines lipophilen nichtionischen grenzflächenaktiven Mittels und eines polyethoxylierten hydrophilen nichtionischen grenzflächenaktiven Mittels im Inneren des Partikels, wobei das Partikel eine Oberfläche aufweist, die einer Vernetzungsbehandlung zum Vernetzen der Oberfläche unterzogen worden ist, wobei das partikelförmige superabsorbierende Polymer eine Vortex-Zeit von 30 bis 60 Sekunden aufweist, wobei der mittlere Porendurchmesser des partikelförmigen superabsorbierenden Polymers in dem Bereich von 10-500 $\mu$m liegt, wobei der mittlere Porendurchmesser gefunden wird, indem der Querschnitt des partikelförmigen superabsorbierenden Polymers in einem trockenen Zustand einer Bildanalyse mithilfe eines Elektronenmikroskops unterzogen wird, wobei der mittlere Porendurchmesser durch Bilden eines Histogramms, das die Verteilung von Porendurchmessern des partikelförmigen superabsorbierenden Polymers als Ergebnis der Bildanalyse darstellt, und Berechnen des anzahlgemittelten Mittelwerts von Porendurchmessern auf Grundlage des Histogramms erhalten wird, wobei das lipophile nichtionische grenzflächenaktive Mittel einen HLB-Wert von 4 bis 9 aufweist und das polyethoxylierte hydrophile nichtionische grenzflächenaktive Mittel einen HLB-Wert von 12 bis 18 aufweist.

**15.** Partikelförmiges superabsorbierendes Polymer gemäß Anspruch 14, wobei das Gemisch eines lipophilen nichtionischen grenzflächenaktiven Mittels und eines polyethoxylierten hydrophilen nichtionischen grenzflächenaktiven Mittels einen HLB-Wert von 8 bis 14 aufweist.

**16.** Partikelförmiges superabsorbierendes Polymer gemäß Anspruch 15, wobei das lipophile nichtionische grenzflächenaktive Mittel ein Sorbitanester ist und das polyethoxylierte hydrophile nichtionische grenzflächenaktive Mittel ein polyethoxylierter Sorbitanester ist.

**17.** Partikelförmiges superabsorbierendes Polymer gemäß Anspruch 14, wobei die partikelförmige superabsorbierende Polymerzusammensetzung Partikel mit Partikeldurchmessern von kleiner als 600 $\mu$m und größer als 150 $\mu$m in einer Menge von nicht weniger als 85 Gew.-% der partikelförmigen superabsorbierenden Polymerzusammensetzung und wie spezifiziert durch eine Standardsieb-Klassierung aufweist und die Partikel einen gewichtsgemittelten Partikeldurchmesser (D50), spezifiziert durch eine Standardsieb-Klassierung, von 300 bis 400 $\mu$m aufweisen.

**18.** Absorptionsfähiger Gegenstand, umfassend:

eine Decklage;
eine rückseitige Lage;
einen absorptionsfähigen Kern, der zwischen der Decklage und der rückseitigen Lage angeordnet ist, wobei der absorptionsfähige Kern eine partikelförmige superabsorbierende Polymerzusammensetzung umfasst, die eine interne Vernetzungsstruktur, von 0,05 bis 2,0 Gew.-%, bezogen auf die Gesamtmenge der Lösung des polymerisierbaren ungesättigten Säuregruppe-enthaltenden Monomers, an einem Schaumbildner und von 0,001 bis 1,0 Gew.-%, bezogen auf die Gesamtmenge der Lösung des polymerisierbaren ungesättigten Säuregruppe-enthaltenden Monomers, an einem Gemisch eines lipophilen nichtionischen grenzflächenaktiven Mittels und eines polyethoxylierten hydrophilen nichtionischen grenzflächenaktiven Mittels im Inneren des Partikels umfasst, wobei das lipophile nichtionische grenzflächenaktive Mittel einen HLB-Wert von 4 bis 9 aufweist und das polyethoxylierte hydrophile nichtionische grenzflächenaktive Mittel einen HLB-Wert von 12 bis 18 aufweist, wobei das Partikel eine Oberfläche aufweist, die einer Vernetzungsbehandlung zum Vernetzen der Oberfläche unterzogen worden ist, wobei das partikelförmige superabsorbierende Polymer eine Vortex-Zeit von 30 bis 60 Sekunden aufweist, wobei der mittlere Porendurchmesser des partikelförmigen superabsorbierenden Polymers in dem Bereich von 10-500 $\mu$m liegt, wobei der mittlere Porendurchmesser gefunden wird, indem der Querschnitt des partikelförmigen superabsorbierenden Polymers in einem trockenen Zustand einer Bildanalyse mithilfe eines Elektronenmikroskops unterzogen wird, wobei der mittlere Porendurchmesser durch Bilden eines Histogramms, das die Verteilung von Porendurchmessern des partikelförmigen superabsorbierenden Polymers als Ergebnis der Bildanalyse darstellt, und Berechnen des anzahlgemittelten Mittelwerts von Porendurchmessern auf Grundlage des Histogramms erhalten wird.

**Revendications**

**1.** Procédé pour la fabrication d'un polymère superabsorbant particulaire ayant une rapide absorption d'eau comprenant les étapes de

a) préparation d'une solution aqueuse de monomères constituée d'un mélange d'un monomère insaturé polymérisable contenant des groupes acides et d'un monomère agent de réticulation interne, la solution aqueuse de monomères comprenant de l'oxygène dissous ;
b) barbotage dans la solution aqueuse de monomères de l'étape a) ce qui inclut l'ajout d'un gaz inerte à la solution aqueuse de monomères de l'étape a) pour remplacer l'oxygène dissous de la solution aqueuse de monomères ;
c) polymérisation de la solution aqueuse de monomères de l'étape b) ce qui inclut les étapes de

c1) ajout à la solution aqueuse de monomères de l'étape a) de : i) une solution aqueuse comprenant de 0,05 à 2,0 % en poids, par rapport à la quantité totale de la solution de monomère insaturé polymérisable contenant des groupes acides, d'un agent moussant ; et ii) une solution aqueuse comprenant de 0,001 à 1,0 % en poids, par rapport à la quantité totale de la solution de monomère insaturé polymérisable contenant des groupes acides, d'un mélange d'un tensioactif lipophile et d'un tensioactif hydrophile polyéthoxylé ;
c2) traitement de la solution de monomères de l'étape c1) par mélange sous cisaillement à grande vitesse pour former une solution de monomères traitée, les composants i) une solution aqueuse comprenant de 0,1 à 1,0 % en poids d'un agent moussant ; et ii) une solution aqueuse comprenant de 0,001 à 1,0 % en poids d'un mélange d'un tensioactif lipophile et d'un tensioactif hydrophile polyéthoxylé étant ajoutés à la solution aqueuse de monomères après l'étape b) de barbotage dans la solution aqueuse de monomères et avant l'étape c2) de mélange sous cisaillement à grande vitesse de la solution aqueuse de monomères ; c3) formation d'un hydrogel par ajout d'un initiateur de polymérisation à la solution de monomères traitée de l'étape c2), l'initiateur étant ajouté à la solution de monomères traitée après l'agent moussant et le mélange de tensioactifs, le polymère étant formé pour inclure des bulles de l'agent moussant dans la structure de polymère ;

d) séchage et broyage de l'hydrogel de l'étape c) pour former du polymère superabsorbant particulaire ; et
e) réticulation en surface du polymère superabsorbant particulaire de l'étape d) avec un agent de réticulation de surface, le polymère superabsorbant réticulé en surface ayant un vortex de 30 s à 60 s, moyennant quoi le polymère superabsorbant inclut de 0, 001 % en poids à 5 % en poids, par rapport à la quantité totale du monomère insaturé polymérisable contenant des groupes acides, d'au moins un agent de réticulation interne, dans lequel le tensioactif lipophile est non ionique et a une HLB de 4 à 9 et le tensioactif hydrophile polyéthoxylé est non ionique et a une HLB de 12 à 18.

2. Procédé pour la fabrication du polymère superabsorbant particulaire selon la revendication 1 dans lequel le mélange d'un tensioactif lipophile et d'un tensioactif hydrophile polyéthoxylé a une HLB de 8 à 14.

3. Procédé pour la fabrication du polymère superabsorbant particulaire selon la revendication 1 dans lequel le tensioactif lipophile est un ester de sorbitane et le tensioactif hydrophile polyéthoxylé est un ester de sorbitane polyéthoxylé.

4. Procédé pour la fabrication du polymère superabsorbant particulaire selon la revendication 1 dans lequel l'agent moussant est choisi entre un carbonate de métal alcalin et un bicarbonate de métal alcalin.

5. Procédé pour la fabrication du polymère superabsorbant particulaire selon la revendication 1 dans lequel le polymère superabsorbant a un indice de capacité d'absorption sous pression de 120 à 150.

6. Procédé pour la fabrication du polymère superabsorbant particulaire selon la revendication 1 comprenant de 0,05 à 1,0 % en poids, par rapport à la quantité totale de la solution de monomère insaturé polymérisable contenant des groupes acides, de l'initiateur de polymérisation.

7. Procédé pour la fabrication du polymère superabsorbant particulaire selon la revendication 1 dans lequel ladite composition de polymère superabsorbant particulaire a des particules ayant des diamètres de particule plus petits que 600 $\mu$m et plus grands que 150 $\mu$m en une quantité de pas moins de 85 % en poids de la composition de polymère superabsorbant particulaire et comme spécifié par classification sur tamis standard et les particules ayant un diamètre de particule moyen en poids (D50) spécifié par classification sur tamis standard de 300 à 400 $\mu$m.

8. Procédé pour la fabrication du polymère superabsorbant particulaire selon la revendication 1 comprenant en outre l'étape de
e) mélange du polymère superabsorbant réticulé en surface avec un agent chélatant,
dans lequel une quantité de l'agent chélatant est de 0,001 à 10 parties en poids pour 100 parties en poids du polymère superabsorbant particulaire.

9. Procédé pour la fabrication du polymère superabsorbant particulaire selon la revendication 8 dans lequel l'agent chélatant est choisi parmi les acides aminocarboxyliques ayant au moins trois groupes carboxyle et leurs sels.

10. Procédé pour la fabrication du polymère superabsorbant particulaire selon la revendication 1 comprenant l'étape d'ajout de 0,01 à 0,5 % en poids d'un polymère thermoplastique par rapport au poids de poudre de polymère sec qui est appliqué sur la surface des particules, le polymère thermoplastique étant soit ajouté au polymère superabsorbant particulaire avec l'agent de réticulation de surface soit appliqué sur le polymère superabsorbant particulaire avant que l'agent de réticulation de surface soit ajouté au polymère superabsorbant particulaire, et de traitement thermique de la particule de polymère superabsorbant revêtue à une température comprise entre 150 °C et 250 °C pendant une durée de 0,5 à 60 minutes pour effectuer la réticulation de surface de la particule de polymère superabsorbant.

**11.** Procédé pour la fabrication du polymère superabsorbant particulaire selon la revendication 10 dans lequel le polymère thermoplastique est choisi parmi du polyéthylène, des polyesters, des polyuréthanes, du polyéthylène basse densité linéaire (PE-BDL), du copolymère d'éthylène et d'acide acrylique (EAA), des copolymères de styrène, du copolymère d'éthylène et de méthacrylate d'alkyle (EMA), du polypropylène (PP), du copolymère d'éthylène et d'acétate de vinyle (EVA) ou des mélanges de ceux-ci, ou des copolymères de ceux-ci.

**12.** Procédé pour la fabrication du polymère superabsorbant particulaire selon la revendication 10 dans lequel le polymère thermoplastique est ajouté au polymère superabsorbant particulaire avec l'agent de réticulation de surface.

**13.** Procédé pour la fabrication du polymère superabsorbant particulaire selon la revendication 10 dans lequel le polymère thermoplastique est ajouté au polymère superabsorbant particulaire avant que l'agent de réticulation de surface c) soit ajouté au polymère superabsorbant particulaire.

**14.** Polymère superabsorbant particulaire comprenant une structure de réticulation interne, de 0,05 à 2 % en poids, par rapport à la quantité totale de la solution de monomère insaturé polymérisable contenant des groupes acides, d'un agent moussant et de 0,001 à 1,0 % en poids, par rapport à la quantité totale de la solution de monomère insaturé polymérisable contenant des groupes acides, d'un mélange d'un tensioactif non ionique lipophile et d'un tensioactif non ionique hydrophile polyéthoxylé dans une partie intérieure de la particule, la particule ayant une surface qui a été soumise à un traitement de réticulation pour la réticulation de la surface, le polymère superabsorbant particulaire ayant une durée de vortex de 30 à 60 secondes,
moyennant quoi le diamètre moyen des pores du polymère superabsorbant particulaire est dans la plage de 10-500 μm, le diamètre moyen des pores étant trouvé par le fait de soumettre la section transversale du polymère superabsorbant particulaire à l'état sec à une analyse d'image à l'aide d'un microscope électronique, le diamètre moyen des pores étant obtenu par formation d'un histogramme représentant la distribution des diamètres des pores du polymère superabsorbant particulaire à la suite de l'analyse d'image et calcul de la moyenne en nombre des diamètres des pores sur la base de l'histogramme,
dans lequel le tensioactif non ionique lipophile a une HLB de 4 à 9 et le tensioactif non ionique hydrophile polyéthoxylé a une HLB de 12 à 18.

**15.** Polymère superabsorbant particulaire selon la revendication 14 dans lequel le mélange d'un tensioactif non ionique lipophile et d'un tensioactif non ionique hydrophile polyéthoxylé a une HLB de 8 à 14.

**16.** Polymère superabsorbant particulaire selon la revendication 15 dans lequel le tensioactif non ionique lipophile est un ester de sorbitane et le tensioactif non ionique hydrophile polyéthoxylé est un ester de sorbitane polyéthoxylé.

**17.** Polymère superabsorbant particulaire selon la revendication 14 dans lequel ladite composition de polymère superabsorbant particulaire a des particules ayant des diamètres de particule plus petits que 600 μm et plus grands que 150 μm en une quantité de pas moins de 85 % en poids de la composition de polymère superabsorbant particulaire et comme spécifié par classification sur tamis standard et les particules ayant un diamètre de particule moyen en poids (D50) spécifié par classification sur tamis standard de 300 à 400 μm.

**18.** Article absorbant comprenant :

une feuille supérieure ;
une feuille arrière ;
une âme absorbante disposée entre la feuille supérieure et la feuille arrière, l'âme absorbante comprenant une composition de polymère superabsorbant particulaire comprenant une structure de réticulation interne, de 0,05 à 2,0 % en poids, par rapport à la quantité totale de la solution de monomère insaturé polymérisable contenant des groupes acides, d'un agent moussant et de 0,001 à 1,0 % en poids, par rapport à la quantité totale de la solution de monomère insaturé polymérisable contenant des groupes acides, d'un mélange d'un tensioactif non ionique lipophile et d'un tensioactif non ionique hydrophile polyéthoxylé dans une partie intérieure de la particule,
dans lequel le tensioactif non ionique lipophile a une HLB de 4 à 9 et le tensioactif non ionique hydrophile polyéthoxylé a une HLB de 12 à 18, la particule ayant une surface qui a été soumise à un traitement de réticulation pour la réticulation de la surface, le polymère superabsorbant particulaire ayant une durée de vortex de 30 à 60 secondes, moyennant quoi le diamètre moyen des pores du polymère superabsorbant particulaire est dans la plage de 10-500 μm, le diamètre moyen des pores étant trouvé par le fait de soumettre la section transversale du polymère superabsorbant particulaire à l'état sec à une analyse d'image à l'aide d'un microscope électronique, le diamètre moyen des pores étant obtenu par formation d'un histogramme représentant la distribution des diamètres des pores du polymère superabsorbant particulaire à la suite de l'analyse d'image et calcul de la moyenne en nombre des diamètres des pores sur la base de l'histogramme.

FIG. 1

FIG. 2

FIG. 3

FIG. 4

FIG. 5

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 02053198 A1
- WO 2010057912 A1
- WO 2013076031 A1
- US 6090875 A
- US 5994440 A